Europäisches Patentamt

European Patent Office

Office européen des brevets

(19)

(11) EP 0 576 439 B1

(12) EUROPEAN PATENT SPECIFICATION

(45) Date of publication and mention
of the grant of the patent:
**06.05.1999 Bulletin 1999/18**

(51) Int Cl.⁶: **C12N 15/13**, C12P 21/08,
C12N 5/20, C12N 15/06,
C07K 14/00, C07K 2/00,
C12N 5/10, G01N 33/577,
G01N 33/569, A61K 39/40

(21) Application number: **92904901.3**

(22) Date of filing: **22.02.1992**

(86) International application number:
**PCT/EP92/00380**

(87) International publication number:
**WO 92/16624 (01.10.1992 Gazette 1992/25)**

(54) **MONOCLONAL ANTIBODY AGAINST LPS CORE**

MONOKLONARER ANTIKÖRPER GEGEN LPS-KERN

ANTICORPS MONOCLONAL DRESSE CONTRE LE NOYAU DE LIPOPOLYSACCHARIDE

(84) Designated Contracting States:
**AT CH DE DK ES FR GB IT LI NL SE**

(30) Priority: **13.03.1991 GB 9105292**

(43) Date of publication of application:
**05.01.1994 Bulletin 1994/01**

(73) Proprietor: **COMMON SERVICES AGENCY**
**Edinburgh EH5 3SE (GB)**

(72) Inventors:
• **GRAM, Hermann**
**D-7858 Weil-Haltingen (DE)**
• **DI PADOVA, Franco**
**CH-4127 Birsfelden (CH)**
• **BARCLAY, George Robin**
**Dalkeith Midlothian EH22 3EL (GB)**
• **POXTON, Ian, Raymond**
**Darkeith Midlothian EH22 3LU (GB)**

(74) Representative: **Horner, Martin Grenville**
**Cruikshank & Fairweather**
**19 Royal Exchange Square**
**Glasgow G1 3AE Scotland (GB)**

(56) References cited:
EP-A- 183 876          EP-A- 239 400
EP-A- 271 379          EP-A- 286 099
EP-A- 341 684          WO-A-84/04458
WO-A-90/03186

• **Nature, vol. 339, no. 6223, 1 June 1989, (London,
GB), V.K. CHAUDHARY et al.: "A recombinant
immunotoxin consisting of two antibody
variable domains fused to Pseudomonas
exotoxin", pages 394-397, see the abstract**
• **Nature, vol. 341, no. 6242, 12 October 1989,
(London, GB), E.S. WARD et al.: "Binding
activities of a repertoire of single
immunoglobulin variable domains secreted
from Escherichia coli", pages 544-546, see the
abstract**
• **Proceedings of the National Academy of
Sciences of USA, vol. 82, no. 6, March 1985,
(Washington, DC, US), N.N.H. TENG et al.:
"Protection against Gram- negative bacteremia
and endotoxemia with human monoclonal IgM
antibodies", pages 1790-1794, see the abstract**
• **Journal of Infectious Diseases, vol. 162, no. 5,
November 1990, (Chicago, IL, US), M. NYS et al.:
"Protective effects of polyclonal sera and of
monoclonal antibodies active to Salmonella
minnesota Re595 lipopolysaccharide during
experimental endotoxemia", pages 1087-1095,
see the abstract**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention relates to the prevention, diagnosis and treatment of infectious diseases caused by Gram-negative bacteria and more particularly provides monoclonal antibodies (MAbs) against the lipopolysaccharide (LPS; also called endotoxin) constituent of the gram-negative bacterial membranes.

Enterobacteria are a widely prevalent group of Gram-negative microorganisms which cause serious and frequently lethal infections in patients undergoing certain types of surgery, anti-cancer chemotherapy or immunosuppressive treatment or in patients suffering from various trauma, burns or wounds. The severity of the disease ranges from a preliminary, transient and limited episode of bacteremia to a subsequent, fulminant and life-threatening conditon of endotoxemia (also called septic shock) characterized, in particular, by a severe hypotension.

Some 425,000 cases of severe Gram-negative bacteremia occur yearly in the USA with an overall mortality of about 25%. The majority of these infections are due to the most common pathogen Escherichia coli, followed in frequency by Klebsiella pneumoniae, Pseudomonas aeruginosa, Proteus, Enterobacter and Serratia. All Gram-negative bacteria are characterized by a specific type of outer membrane which comprises a lipopolysaccharide (LPS) as major constituent. LPS plays an essential immunologic and physiopathologic role in the infections and is the major causative agent of septic shock.

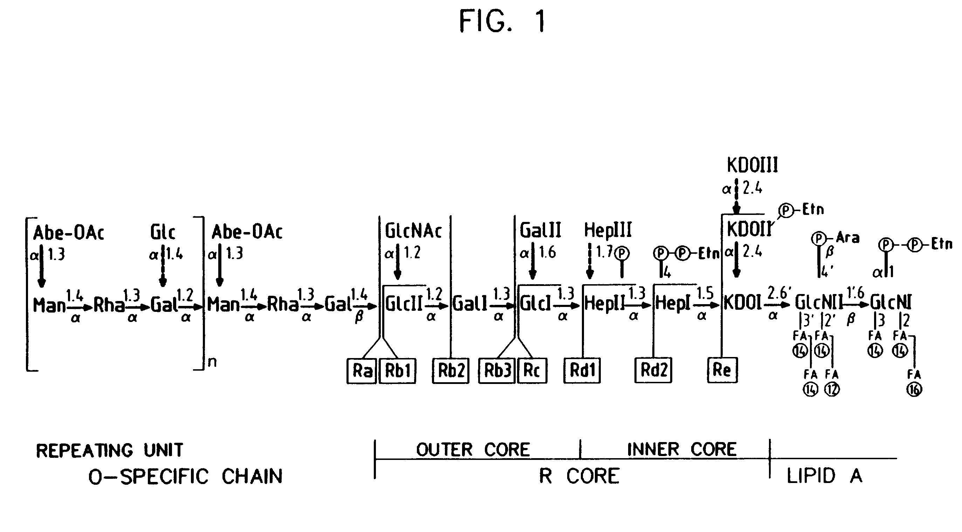

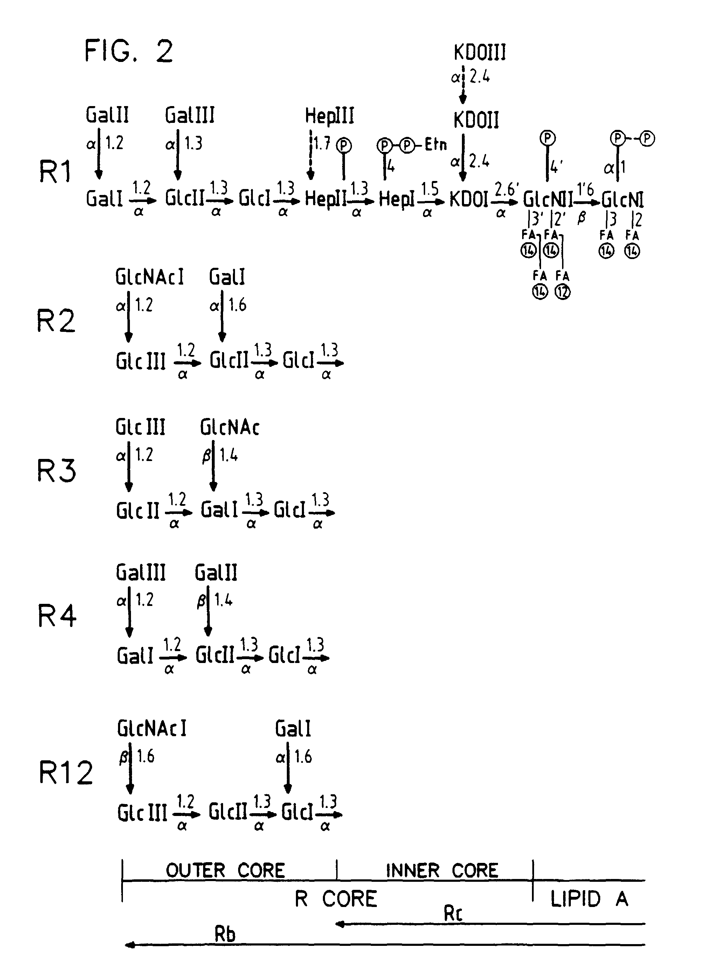

Although the LPS constituent varies from one species to another, it may be generally described with reference to Figure 1 as consisting of three structural regions: Lipid A whose lipid portion is embedded in the outer leaflet of the outer membrane; the oligosaccharide core region and the 0-specific outer region. Lipid A has the same basic structure in practically all enterobacteria and is the main endotoxic determinant. The core region shows a high degree of similarity among bacterial genera. It usually consists of a limited number of sugars. The inner core region is constituted of heptose and 2-Keto-3-deoxyoctonate (KD0) residues while the outer core region comprises galactose, glucose or N-acetyl-D-glucosamine residues displayed in various manners, depending upon the strain. For example, outer core structures R1 to R4 of different E. coli strains are shown in Figure 2. The 0-specific outer region (also called 0-specific side chain) is highly variable and is composed of repeated oligosaccharide units characteristic of the species. LPS molecules on the surface of a single cell do not have a constant amount of oligosaccharide units.

The presence of the 0-specific side chain confers to a culture of a wild type bacterium a smooth aspect. This is the reason why wild type bacteria are usually referred to as smooth bacteria in contrast with rough mutants which lack the 0-specific side chain and, sometimes, part of the core region and the cultures of which show a rough aspect. The different types of rough mutants from Salmonella are conventionally designated by the terms Ra, Rb, Rc, Rd and Re. As seen from Figure 1, the LPS of all of them comprises the lipid A structure while the Ra mutant is characterised by a complete core region, the Rb mutant is characterised by the absence of N-acetyl-D-glucosamine residue, the Rc mutant is characterised by the absence of N-acetyl-D-glucosamine and galactose residues, the Rd mutant is characterised by the absence of any residue constituting the outer core and the Re mutant is characterised by the sole KD0 region attached to lipid A.

Since treatments for the toxic effect of LPS are not available, attention has been focused on immunologic methods as an alternative or additional treatment to antibiotic therapy to prevent or control such infections. Current immunotherapy involves the administration of conventional polyclonal antisera and hyperimmune sera to bolster the native defenses of patients against the adverse effects of bacteria, for example, by enhancing opsonization and phagocytosis of the bacterial cells or by neutralization of the biological activity of LPS. However, the effectiveness of the antisera greatly varies depending upon a large number of factors including, for example, the composition and titre of the specific antibodies, which cannot be easily standardized.

To overcome the limited efficacy of serotherapy, it has been proposed to use cross-reactive MAbs. Cross-reactivity is of two kinds, which may be described as horizontal and vertical. By vertical cross-reactivity is meant that the MAb reacts with essentially all smooth LPS molecules of a particular bacterial strain, independent of the length of the 0-specific side-chain. By horizontal cross-reactivity is meant that the MAb reacts with LPS having different core structures. This is necessary because therapy must be started as soon as the bacteremia has been empirically diagnosed, rather than waiting for the identification of the pathogen, which may take several days.

Such MAbs must recognize antigenic determinants located in the LPS structure which is shared by most enterobacteria i.e. Lipid A and the core region. They may be obtained by the well-known Kohler & Milstein method which, in particular comprises conventionally immunizing mice with an immunogen in which the inner antigenic epitopes of LPS are immediately available for raising antibodies. Suitable immunogens include heat-killed rough mutants of an enterobacterium e.g. the J5 strain of E. coli. Purified LPS is less suitable as an immunogen.

A MAb expected to be useful for preventing or treating bacteremia should not only be cross-reactive but also cross-protective against the infections caused by the most common toxic bacteria. However, it has been reported in several scientific articles, for example, in Pollack et al. J. Infect. Dis. (1989) 159 (2): 168, that the large majority of antibodies raised against the conventional immunogens cited above cross-react poorly and, unfortunately fail to be protective against infections. MAbs have often been described as reactive on the basis of binding experiments involving rough rather than smooth LPS, and the lack of protectivity of these MAbs may be due to the fact that, in wild-type smooth LPS, the epitope for which the antibody is specific is not available, being hindered by the core region or the O-specific side chain. In particular, MAbs recognizing epitopes in the Lipid A part of the LPS molecule are generally ineffective.

It has now been found that monoclonal antibodies recognizing epitopes in the core region of the LPS molecule and having both vertical and horizontal cross-reactivity and also cross-protectivity can be obtained by modified and improved immunization and screening procedures. Such MAbs are initially obtained in murine form and are converted by known recombinant DNA techniques into chimeric (murine variable region, human constant region) or humanized (murine hypervariable regions, human framework and constant region) forms.

Accordingly the present invention provides a monoclonal antibody which recognizes an epitope in the core region of the LPS molecule and which is cross-protective against endotoxemia caused by at least two different Gram-negative bacterial strains having different core structures, said monoclonal antibody having a constant region of human origin, a variable framework region of human or nonhuman origin, and a hypervariable region of nonhuman origin.

Preferably the MAb recognises an epitope which is already present in the Rc core structure of E. coli and is also present in the complete core.

In E. Coli, the MAb of the invention preferably reacts with all common smooth strain isolates, and preferably also with rough strain mutants of all five core types (R1, R2, R3, R4, and K12). Preferably the MAb is also reactive with different strains of Salmonella.

In contrast to the immunization protocols described in the prior art, in which generally a single type of LPS (normally as heat-killed bacteria bearing the specific type of LPS) is used as immunogen, MAbs of the present invention may be produced by an immunization protocoll in which the animal to be immunized is exposed to a plurality of types of LPS molecule. This may be done either by immunization with a cocktail of different LPS types physically mixed together, or by immunizing in sequence by individual different LPS types. In both cases it is prefered to use heat-killed bacteria rather than purified LPS molecules. Other possible immunogens include bacteria killed by means other than heat (e.g. by formaldehyde) and LPS molecules linked to protein carriers.

The animal to be immunized is preferably a mouse, which may be of the Balb-c strain. It may however be preferable to use mice of different genetic background, for example New Zealand Black or Swiss Webster mice, which are capable of giving a wider immune response. The immunogen may be administered intravenously, or, preferably, subcutaneously, for example in the foot pad.

In a first preferred method, mice are immunized with a single cocktail of different strains of heat-killed bacteria, preferably rough strains having a complete core, for example a mixture of R1, R2, R3 and R4 strains of Ra E. coli. Alternatively two or more such cocktails, which may be different, may be given on different occasions. For example, injection of a mixture of E. coli R2 and R3 and Salmonella minnesota R60 may be followed a week later by a mixture of E. coli R1, R4 and 018 rough strain, and then the two injections repeated at further weekly intervals.

In a second preferred method, mice are immunized sequentially with a number of different rough strains of heat-killed bacteria, only one strain being administered at any one time. For example mice may be immunized with Pseudomonas PAC 605 rough mutant followed by E. coli R1, R2 and R3 at monthly intervals.

Before any cell fusion is carried out between mouse myeloma cells and spleen cells from the immunized animal, there is preferably an initial screening step in which the strength and plurality of the immune response of the immunized animal is evaluated by testing the serum of the animal. Animals showing a strong immune response are subjected to a booster immunization and the spleen cells of these strongly-responding and re-immunized mice are used for cell fusion to make hybridomas by the conventional Köhler-Milstein technique. The booster immunization is preferably by a cocktail of different rough strain E. coli, even if the primary immunization was carried out by the second preferred method (sequential administration).

The resulting hybridomas are then screened for the cross-reactivity of the antibodies they produce, using the standard ELISA and Western blotting methods described below. In contrast to prior art methods, an initial screening is preferably carried out using a series of mixtures of different smooth and rough LPS types to select those MAbs reacting with a wide range of LPS molecules. In this way, widely cross-reactive MAbs can already be identified at the initial screening stage. For example, each hybridoma supernatant may be screened by testing for reactivity in the ELISA assay with seven different LPS cocktails and a control, according to the following scheme:

    1) Smooth strains: Ec04 + 06 + 016 + 018K
    2) Smooth strains: Ec012 + 015 + 086
    3) Rough complete core: EcR1 + R4
    4) Rough complete core: EcR2 + EcR3 + EcK12 + Sm R60
    5) Rc core: Ec J5 + St878
    6) Rc/Rd/Re core: Sm R5 + Sm R7 + Sm R4 + Ec F515 + Sm R595
    7) Lipid A : derived from Ec K12 & Sm R595

8) Negative control: BSA

(Ec = E. coli, Sm = Salmonella minnesota, St = S. typhimurium, BSA = bovine serum albumin)

MAbs found to have good cross-reactivity are then screened further to select those which are not only cross-reactive, but also cross-protective.

This may be done using the following in vitro bioassay:

Inhibition of LPS-induced IL-6 secretion by murine peritoneal macrophages

Several monokines including Tumor Necrosis Factor (TNF), IL-1 and IL-6 (also called Interferon-β2) mediate many of the pathophysiological events associated with gram-negative sepsis and its accompanying endotoxemia. These monokines are secreted by macrophages, both in vitro and in vivo, in response to LPS. A protective anti-LPS antibody blocks the macrophage stimulation as shown in the following assay:

Murine peritoneal cells are obtained by peritoneal lavage with 0.34 M sucrose in distilled water. Peritoneal cells are seeded at $5.10^5$ cells/ml in 0.2 ml serum free medium (IMDM-ATL, Schreier and Tees, Immunological Methods, Vol. II, Acad. Press (1981):263) and cultured for 4 hrs at 37° C (i) in the presence or absence of LPS e.g. LPS from E. coli R1 (0.05 ng/ml); E. coli R2 (0.05 ng/ml); E. coli R3 (0.05 ng/ml) and E. coli R4 (0.05 ng/ml); and (ii) in the presence or absence of a purified, endotoxin-free antibody the final concentration of which ranges from 0.05 ng to 50 µg/ml. The supernatants are recovered and the amount of IL-6 present in the supernatants is then measured using the IL-6 dependent hybridoma cell-line B13.29 (Aarden et al., Eur. J. Immunol. 1987, 17, 1911) as follows:

B13.29 cells are seeded at $2.5 \times 10^4$ cells/ml in serum free medium and cultured for 72 hrs in the absence of IL-6 and in the presence or absence of culture supernatant. Aliquots of the cultures (200 µl/well) are distributed in flat bottomed microtitre plates. IL-6 concentration in the supernatants is calculated in relation to a standard curve of IL-6.

For the purposes of this patent specification, a MAb is regarded as being protective against a given LPS if it gives in the above assay a reduction of IL-6 secretion of at least 50X when tested at a concentration of 5 µg/ml, the concentration of purified LPS being 0.05 ng/ml for rough LPS and correspondingly higher for the less active smooth types. A MAb is cross-protective if it is protective against at least two LPS having different core structures. Preferred cross-protective MAbs are cross-protective against LPS from different bacterial genera.

Prefered MAbs of the invention are of the IgG isotype.

By the use of the above immunization and screening methods, a number of novel mouse anti-LPS antibodies have been found which cross-react with several LPS of different genera and exhibit substantial cross-protective activity and that it is possible to construct other LPS binding molecules derived from these monoclonal antibodies and having the same characteristics since they share regions which determine the binding specificity i.e. the hypervariable regions. In particular, four preferred murine monoclonals according to the invention are hereinafter designated WN1 222-5 (isotype IgG2a), WN1 58-9 (IgG2b), H1 61-2 (IgG1), and SZ27 19.16.07 (IgG2a). Of these, the first two are particularly preferred.

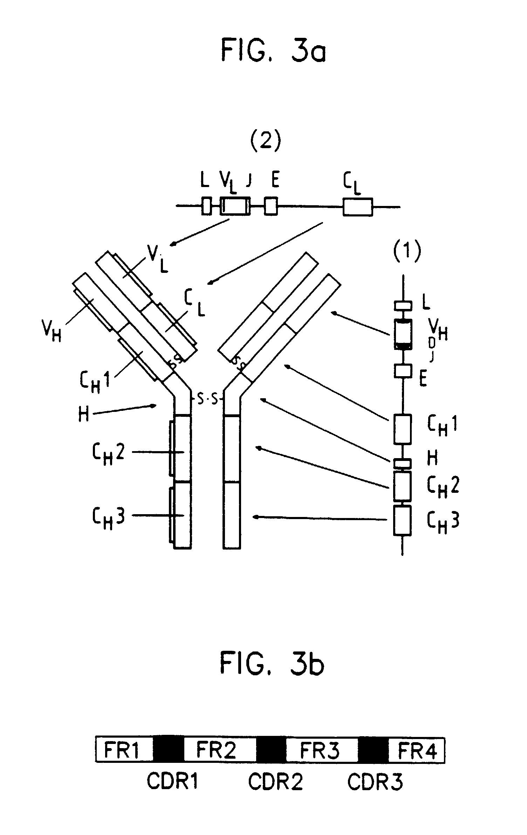

Natural immunoglobulins or antibodies comprise a generally Y-shaped molecule built up of two identical heavy chains and two identical light chains, and having an antigen-binding site at the end of each upper arm. The remainder of the structure, in particular the stem of the Y, mediates effector functions associated with the immunoglobulins. The general structure of an antibody of the IgG class is shown schematically in Figure 3A. Both heavy and light chains comprise a variable domain and a constant part. An antigen binding site consists of the variable domain of a heavy chain ($V_H$) associated with the variable domain of a light chain ($V_L$). The variable domains of the heavy and light chains have the same general structure which is illustrated in Figure 3B.

More particularly, the antigen binding characteristics of an antibody are essentially determined by 3 specific regions in the variable domain of the heavy and light chains which are called hypervariable regions or complementary determining regions (CDRs). As shown in Figure 3B, these 3 hypervariable regions alternate with 4 framework regions, (FRs) whose sequences are relatively conserved and which are not directly involved in binding. The CDRs form loops and are held in close proximity by the framework regions which largely adopt a β-sheet conformation. The CDRs of a heavy chain together with the CDRs of the associated light chain essentially constitute each of the two antigen binding sites of the antibody molecule.

The determination as to what constitutes a FR or a CDR region is usually made by comparing the amino acid sequence of a number of antibodies raised in the same species. The general rules for identifying the CDR and FR regions are given in Table I.

Furthermore, it has been recently found that the contribution made by a light chain variable domain to the energetics of binding is small compared to that made by the associated heavy chain variable domain and that isolated heavy chain variable domains have an antigen binding activity of their own. Such molecules, now commonly referred to as single domain antibodies, may be regarded as having an antigen binding site, even in the absence of an associated $V_L$ domain.

In view of the foregoing, the invention provides a LPS binding molecule which comprises at least one antigen binding site comprising at least one domain which comprises in sequence, the hypervariable regions hCDR1, hCDR2 and hCDR3 ; (domains h222-5 and h58-9) said hCDR1 having the amino acid sequence Asp Tyr Tyr Met Thr; said hCDR2 having the amino acid sequence Leu Ile Arg Asn W Arg Asn Gly Asp Thr Ala Glu Tyr Ser Ala Ser Val X;
wherein W is Lys or Tyr and X is Lys or Arg;
said hCDR3 having the amino acid sequence Gln Gly Arg Gly Tyr Thr Leu Asp Tyr;
and direct equivalents thereof.

Preferred is the hypervariable region hCDR2 in which W is Lys and X is Lys (h222-5) or in which W is Tyr and X is Arg (h58-9). More preferred is the hypervariable region hCDR2 in which W is Lys and X is Lys.

In a first aspect of the invention, the LPS binding molecule comprises an antigen binding site comprising a single domain.

In a second aspect of the invention, the LPS binding molecule comprises at least one antigen binding site comprising:

a) a first domain comprising in sequence the hypervariable regions hCDR1, hCDR2 and hCDR3, as defined above and,

b) a second domain comprising in sequence the hypervariable regions lCDR1, lCDR2 and lCDR3; (domains l222-5 and l58-9 [l222-5 or l58-9 stands for light 222-5 or light 58-9]) said lCDR1 having the amino acid sequence Arg Ala Y Z Asn Ile Asn
Ile Trp Leu Ser;
wherein Y is Ser or Arg and Z is Gln or Leu;
said lCDR2 having the amino acid sequence Lys Ala Ser Asn Leu His Thr;
said lCDR3 having the amino acid sequence Leu Gln Gly Gln Ser Tyr Pro Arg Thr;
and direct equivalents thereof.

Preferred is the hypervariable region lCDR1 in which Y is Ser and Z is Gln (l222-5) or in which Y is Arg and Z is Leu (l58-9). More preferred is the hypervariable region lCDR1 in which Y is Ser and Z is Gln.

Unless otherwise indicated, any polypeptide chain is hereinafter described as having an amino acid sequence starting at the N-terminal extremity and ending at the C-terminal extremity.

When the antigen binding site comprises both the first and second domains, these may be located on the same polypeptide molecule or, preferably, each domain may be on a different chain, the first domain being part of an immunoglobulin heavy chain or fragment thereof and the second domain being part of an immunoglobulin light chain or fragment thereof.

By "LPS binding molecule" is meant any molecule capable of binding to LPS. The binding reaction may be shown by standard methods (qualitative assays) such as an ELISA using purified LPS or heat treated bacteria or a Western blotting using purified LPS; with reference to a negative control test in which an antigen of unrelated origin, e.g. bovine serum albumin (BSA), is used. A complete description of the assays cited above is given below.

1. Detection of binding to purified LPS in an ELISA

Microtitre plates (flat bottomed; microtest III flexible assay plates; Becton Dickinson, Falcon 3912) are coated with purified LPS at 2 µg/ml in coating buffer (diethylenebarbituric acid-Na salt 30 mM, Na acetate 30 mM, NaCl 116 mM; pH 4.5). 50 µl aliquots of the LPS solution are distributed into each well. Unrelated protein (BSA, 2% in PBS pH 7.2/0.02% sodium azide) is used to determine non-specific binding. Plates are incubated for 1 hr at 37°C and then overnight at 4°C in a humidified chamber. Plates are washed 4 times with a washing solution i.e. phosphate buffered saline (PBS) pH 7.2, 0.05% vol/vol Tween 20, 0.02% sodium azide. Plates are blocked with 250 µl/well of 2% BSA in PBS/sodium azide 0.02% for 3 hrs at room temperature. Plates are washed again.

Antibody solutions are prepared in PBS/BSA 2%/sodium azide 0.02% at various dilutions, e.g. 1 ug/ml, 100 ng/ml, 10 ng/ml and 1 ng/ml. 50 µl aliquots of these solutions are distributed in the wells of the precoated plates. Incubation is carried out overnight at room temperature. After 4 washes, 50 µl per well of biotinylated affinity purified goat anti-mouse IgG or IgM of the correct subclass specificity, e.g. anti-mouse IgG2a for WN1 222-5 and anti-mouse IgG2b for WN1 58-9 or anti-human IgG1 or IgM for a variation of WN1 222-5 $_{(huIgM)}$ or WN1 222-5$_{(huIgG1)}$ (final dilution 1/10'000 in PBS 2% BSA; Southern Biotechnology Associates) is added. Incubation is carried out for 4 hrs at room temperature. After 4 washes, 50 µl per well of streptavidin alkaline phosphatase conjugate (Jackson Immuno Research Laboratories; final dilution 1/10'000 in PBS, 2X BSA) is added; Incubation is carried out for 1 hr at room temperature. After 4 washes, 100 µl per well of paranitrophenol phosphate (PNPP) diluted at 1 mg/ml in diethanolamine buffer (diethanolamine 1M, HgCl$_2$.6H$_2$O 0.5 mM, pH 9.8) is added. After 1 hr, absorbance is read at 405 nm using a Titertek Multiskan ELISA reader (MCC/340, Flowlabs).

Advantageously, the purified LPS which is used is selected from smooth, complete core, Rb or Rc LPS. Examples of smooth LPS are LPS extracted from E. coli 0111B4 (Difco), E. coli 0127B8 (Difco), E. coli 0128B12 (Difco), Salmonella typhimurium B0 ag 0:4, 5, 12 (SH 4809) (Bio-carb). Suitable complete core LPS, Rb LPS and Rc LPS are respectively obtained from S. minnesota (List) and S. typhimurium SL 684 (Sigma).

Tables IIA, IIB, IIC, and IID show in tabular form the binding of antibodies WN1 222-5, WN1 58-9, H1 61-2 and SZ27 19.16.07 respectively to purified LPS from different strains of Gram-negative bacteria.

## 2. Detection of binding to heat killed bacteria

Precoated plates are prepared as described in 1. above, using heat killed bacteria ($0.5 \times 10^8$ cells/ml) rather than purified LPS. The binding reaction is tested and detected as described in 1. above.

Advantageously, the bacteria are smooth wild type bacteria or rough Ra, Rb or Rc mutants.

Tables IIIA, IIIB, IIIC, and IIID show in tabular form the binding of antibodies WN1 222-5, WN1 58-9, H1 61-2 and SZ27 19.16.07 respectively to heat killed bacteria of various Gram-negative strains.

The bacteria listed in Tables II and III are mostly common clinical isolates. The bacteria and/or the corresponding LPS are commercially available or are available on request from Dr. I. Poxton, Dept. of Bacteriology, University of Edinburgh, Scotland, or from Dr H. Brade, Forschungsinstitut Borstel, Borstel, W. Germany.

As will be seen from Tables II and III, the minimum core structure required for recognition by the antibodies of the invention is Rc.

## 3. Detection of binding to LPS using Western blotting

10 μl aliquots of a LPS solution at 1 mg/ml are mixed with an equal volume of 0.1 M Tris-HCl buffer, pH 6.8 containing 1% (wt/vol) sodium deoxycholate (DOC), 20% (wt/vol) glycerin and 0.001% bromophenol blue, and then sonicated. The samples so prepared are loaded onto an electrophoresis gel (4% stacking gel; 14% running gel). The electrophoresis system which is used is a modified Laemmli system (DOC-PAGE; Komuro et al Chem. Pharm. Bull. (1988) 36: 1218) using a Mini Protean II dual slab cell apparatus (Bio Rad Laboratories). The samples are run at a current of 18 mA until the indicator dye enters the separating gel. The current is then increased to 25 mA.

Blotting of the gel is carried out using a 0.45 μm pore size nitrocellulose membrane (Bio Rad Laboratories) and a transfer electrophoresis cell (Mini transblot electrophoretic transfer cell apparatus, Bio Rad Laboratories) at 60 V for 20 min. The blot is soaked in Tris buffer saline (TBS: 20 nM Tris-HCl, 0.1 mM NaCl; pH 7.5) 1% BSA for 1 hr at room temperature. The immunoblot is developed for 2 hrs at room temperature using an antibody preparation at 0.1 μg/ml in TTBS (TBS, 0.05% Tween 20) 1% BSA.

The blot is washed twice in TTBS and further incubated for 45 min at room temperature with a biotinylated goat anti-mouse IgG2a or IgG2b antibody (Southern Biotechnology associates) at a final dilution of 1/10'000 in TTBS, 1% BSA. After washing twice, streptavidin alkaline phosphatase conjugate (Jackson Immuno Research Laboratories), used at a dilution of 1/10'000 in TTBS/BSA 1%, is added. Incubation is carried out for 45 min at room temperature. After 3 washes, the BCIP/NBT alkaline phosphatase colour development solution is added as indicated by the manufacturer (Bio Rad Laboratories). In parallel, the gel is fixed by overnight incubation in a solution containing 40X ethanol and 5% acetic acid and is silver-stained according to the method of Tsai and Frash, Ann. Biochem. (1982) 119: 115.

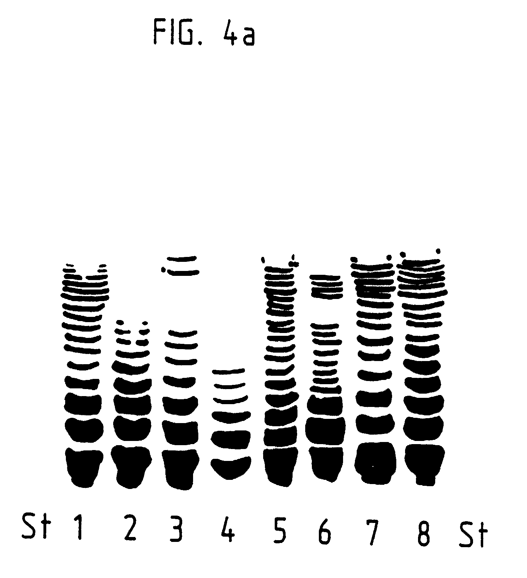

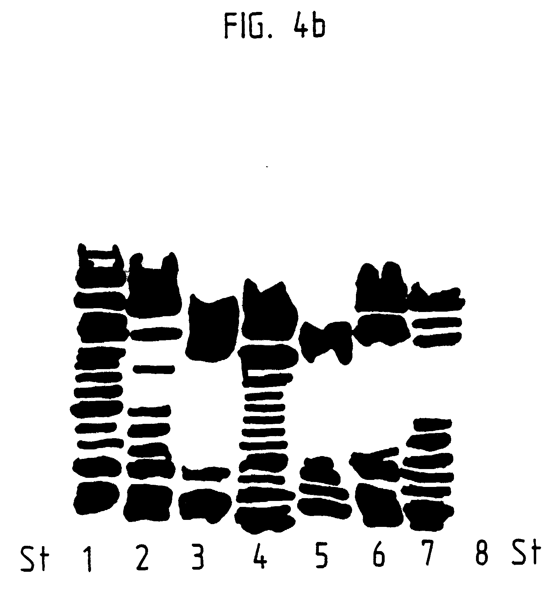

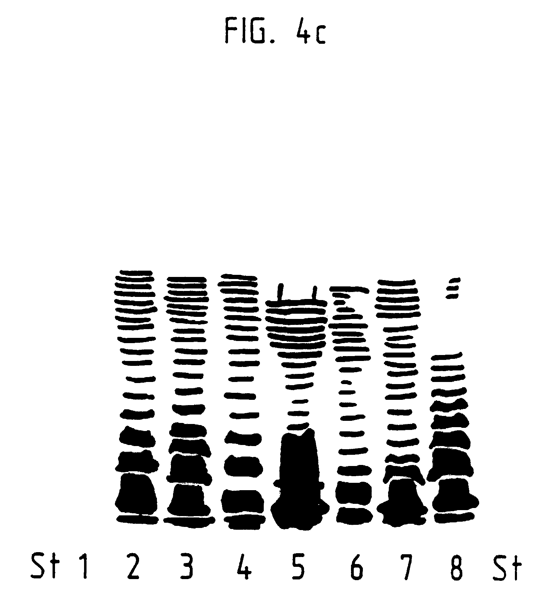

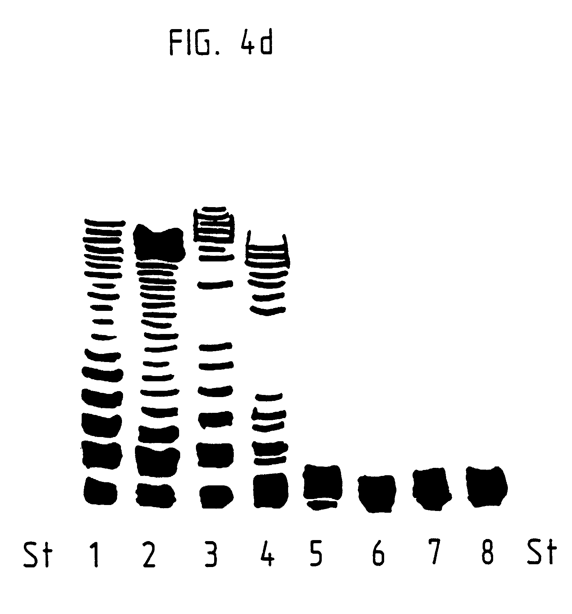

In this assay, the antibodies of the invention show a binding reaction with LPS extracted either from smooth bacteria or from rough mutants. Particular experiments involving WN1 222-5, WN1 58-9, H1 61-2 and SZ27 19.16.07 are to be seen in Figures 4A; 4B, 4C and 4D respectively. The LPS content extracted from a smooth bacterium is separated by electrophoresis into bands corresponding to LPS molecules having different molecular weights, depending on the size of the 0-specific side chain. These LPS molecules range from LPS molecules without any 0-specific side chain to LPS molecules having 40 or more units in the side chain. The antibodies of the invention react with rough repeating units and all these LPS molecules, containing 0-side chain repeating units. This indicates that the epitope for which the LPS-binding molecules of the invention are specific is not hindered by the O-specific side chain. Therefore the majority of LPS molecules of a smooth bacterium are able to react with an LPS-binding molecule of the invention.

Examples of antigen binding molecules include immunoglobulin (Ig) molecules, e.g. antibodies as produced by B-cells or hybridomas and chimeric or humanized antibodies or fragments thereof, e.g. F(ab')$_2$ and Fab fragments, as well as single chain or single domain antibodies. Immunoglobulin molecules may be of different isotypes, for example IgG, IgM, IgA or IgE antibodies, of which IgG are preferred.

A single chain antibody consists of the variable domains of the antibody heavy and light chains of an Ig molecule covalently bound by a peptide linker usually consisting of from 10 to 30 amino acids, preferably from 15 to 25 amino acids. Therefore, such a structure does not include the constant part of the heavy and light chains and it is believed that the small peptide spacer is less antigenic than a whole constant part. By "chimeric antibody" is meant an antibody in which the constant regions of the heavy or light chain or both are of human origin while the variable domains of both heavy and light chains are of non-human (e.g. murine) origin. By "humanized antibody" is meant an antibody in which the hypervariable regions are of non-human (e.g. murine) origin, while all other parts of the immunoglobulin molecule, i.e. the constant regions and the highly conserved framework regions of the variable domains, are of human origin.

Hypervariable regions may be associated with any kind of framework regions, preferably of murine or human origin. Suitable framework regions are described in "Sequences of proteins of immunological interest", Kabat E.A. et al, US department of health and human services, Public health service, National institute of health. However, the preferred framework regions are those of WN1 222-5 or WN1 58-9, wherein the regions of WN1 222-5 are the most preferred.

Sequence Identifier No. 1 shows the complete amino acid sequence of the heavy chain variable domain of WN1 222-5, which consists, in sequence from the N-terminal, of framework regions hFR1, hFR2, hFR3 and hFR4 interspersed with the hypervariable regions hCDR1, hCDR2 and hCDR3, whose amino acid sequence is also stated above. In hCDR2 of WN1 222-5 W stands for Lys and X stands for Lys. Sequence Identifier No. 2 shows the complete amino acid sequence of the heavy chain variable domain of WN1 58-9, which consists, in sequence from the N-terminal, of framework regions hFR1$_r$; hFR2$_r$; hFR3$_r$ and hFR4 interspersed with the hypervariable regions hCDR1; hCDR2 and hCDR3, whose amino acid sequence is also stated above. In hCDR2 of WN1 58-9 W stands for Tyr and X stands for Arg. The index r after the FR stands for an amino acid sequence nearly identical to the amino acid sequence without index. The sequence with index comprises at least one replaced amino acid in contrast to the sequence without index.

Sequence Identifier No. 3 shows the complete amino acid sequence of the light chain variable domain of WN1 222-5, consisting in sequence of framework regions IFR1; IFR2; IFR3 and IFR4 interspersed with the hyper- variable regions ICDR1; ICDR2 and ICDR3 whose amino acid sequence is also stated above. In ICDR1 of WN1 222-5 Y stands for Ser and Z stands for Gln. Sequence Identifier No. 4 shows the complete amino acid sequence of the light chain variable domain of WN1 58-9, consisting in sequence of framework regions IFR1$_r$; IFR2$_r$; IFR3$_r$ and IFR4 interspersed with the hypervariable regions ICDR1, ICDR2 and ICDR3 whose amino acid sequence is also stated above. In ICDR1 of WN1 58-9 Y stands for Arg and Z stands for Leu.

The preferred heavy chain framework is hFR1; hFR2; hFR3 and hFR4 as shown in Seq. Id. No. 1 and the preferred light chain framework is IfR1; IFR2; 1FR3 and IFR4 as shown in Seq. Id. No. 3.

Accordingly, the invention also provides an LPS binding molecule which comprises at least one antigen binding site comprising either a domain having an amino acid sequence substantially identical to that shown in Seq. Id. No. 1 or alternatively No. 2 starting with amino acid at position 1 and ending with amino acid at position 120; or a first domain as described above and a second domain having an amino acid sequence substantially identical to that shown in Seq. Id. No. 3 or alternatively 4, starting with amino acid at position 1 and ending with amino acid at position 107.

Monoclonal antibodies raised against a toxic antigen must necessarily be developed in a non-human system e.g. in mice. As a direct consequence of this, a xenogenic antibody as produced by a hybridoma, when administered to humans, elicits an undesirable immune response which is essentially mediated by the constant part of the xenogenic immunoglobulin. This clearly limits the use of such antibodies as they cannot be administered over a prolonged period of time. Therefore it is particularly preferred to use single chain antibodies or chimeric or humanized monoclonal antibodies which are less likely to elicit a substantial allogenic response when administered to humans.

In view of the foregoing, a more preferred LPS binding molecule of the invention is selected from a chimeric anti-LPS antibody which comprises at least

a) one immunoglobulin heavy chain or fragment thereof which comprises

(i) a variable domain comprising in sequence the hypervariable regions hCDR1, hCDR2 and hCDR3 as shown in Seq. Id. No. 1 or No. 2 and
(ii) the constant part or fragment thereof of a human heavy chain; and,

b) one immunoglobulin light chain or fragment thereof which comprises

(i) a variable domain comprising in sequence the hypervariable regions ICDR1; ICDR2 and ICDR3 as shown in Seq. Id. No. 3 or No. 4 and
(ii) the constant part or fragment thereof of a human light chain;

and direct equivalents thereof.

Alternatively, a LPS binding molecule of the invention may be selected from a single chain binding molecule which comprises an antigen binding site comprising

a) a first domain comprising in sequence the hypervariable regions hCDR1, hCDR2 and hCDR3, as shown in Seq. Id. No. 1 or No. 2,

b) A second domain comprising in sequence the hypervariable regions lCDR1, lCDR2 and lCDR3, as shown in Seq. Id. No. 3 or No. 4 and

c) a peptide linker which is bound either to the N-terminal extremity of the first domain and to the C-terminal extremity of the second domain or to the C-terminal extremity of the first domain and to the N-terminal extremity of second domain;

and direct equivalents thereof.

As is well known, minor changes in an amino acid sequence such as deletion, addition or substitution of one or several amino acids may lead to an allelic form of the original protein which has substantially identical properties. Thus, by the term "direct equivalents thereof" is meant either any single domain LPS binding molecule (molecule X)

(i) in which the hypervariable regions taken as a whole are at least 80% homologous, preferably at least 90% homologous, more preferably at least 95% homologous to the hypervariable regions hCDR1, hCDR2 and hCDR3 as shown in Seq. Id. No. 1 or 2 and,

(ii) which is capable of binding to LPS substantially to the same extent as a reference molecule having framework regions identical to those of molecule X but having hypervariable regions hCDR1, hCDR2 and hCDR3 identical to those shown in Seq. Id. No. 1 or No. 2;

or any LPS binding molecule having at least two domains per binding site (molecule X')

(i) in which the hypervariable regions taken as a whole are at least 80% homologous, preferably at least 90% homologous, more preferably at least 95% homologous to the hypervariable regions hCDR1, hCDR2, hCDR3, lCDR1, lCDR2 and lCDR3 as shown in Seq. Id. No. 1; 2; 3 and 4, and

(ii) which is capable of binding to LPS substantially to the same extent as a reference molecule having framework regions and constant parts identical to molecule X' but having hypervariable regions hCDR1, hCDR2, hCDR3, lCDR1, lCDR2 and lCDR3 identical to those shown in Seq. Id. No. 1; 2; 3 and 4.

One LPS binding molecule may be considered as binding to LPS substantially to the same extent as another if the two molecules can be shown effectively to compete with each other in competitive ELISA binding assays on different LPS molecules, for example on the LPS from E. coli J5 and from Salmonella Ra 60 and if the binding affinities of the two molecules vary from each other in each case by a factor of not more than 100, preferably not more than 10.

Most preferably, the chimeric anti-LPS antibody comprises at least

a) one heavy chain which comprises a variable domain having an amino acid sequence substantially identical to that shown in Seq. Id. No. 1 starting with amino acid at position 1 and ending with amino acid at position 120 and the constant part of a human heavy chain; and

b) one light chain which comprises a variable domain having an amino acid sequence substantially identical to that shown in Seq. Id. No. 3 starting with amino acid at position 1 and ending with amino acid at position 107 and the constant part of a human light chain.

The constant part of a human heavy chain may be of the $\gamma_1$, $\gamma_2$, $\gamma_3$, $\gamma_4$, $\mu$, $\alpha_1$, $\alpha_2$, $\delta$ or $\epsilon$ type, preferably of the $\gamma$ type, more preferably of the $\gamma_1$ type, whereas the constant part of a human light chain may be of the $\kappa$ or $\lambda$ type, preferably of the $\kappa$ type. The amino acid sequence of all these constant parts are given in Kabat et al. (supra).

Conjugates of the LPS binding molecules of the invention, e.g. enzyme or toxin conjugates, are also included within the scope of the invention, as are LPS binding molecules labelled with radioactive isotopes or fluorescent markers.

A LPS binding molecule of the invention may be produced by recombinant DNA techniques. In view of this, one or more DNA molecules encoding the binding molecule must be constructed, placed under appropriate control sequences and transferred into a suitable host organism for expression.

In a very general manner, there are accordingly provided

(i) DNA molecules encoding a single domain LPS binding molecule of the invention, a single chain LPS binding molecule of the invention, a heavy or light chain or fragment thereof of a LPS binding molecule of the invention and

(ii) the use of the DNA molecules of the invention for the production of a LPS binding molecule of the invention by recombinant means.

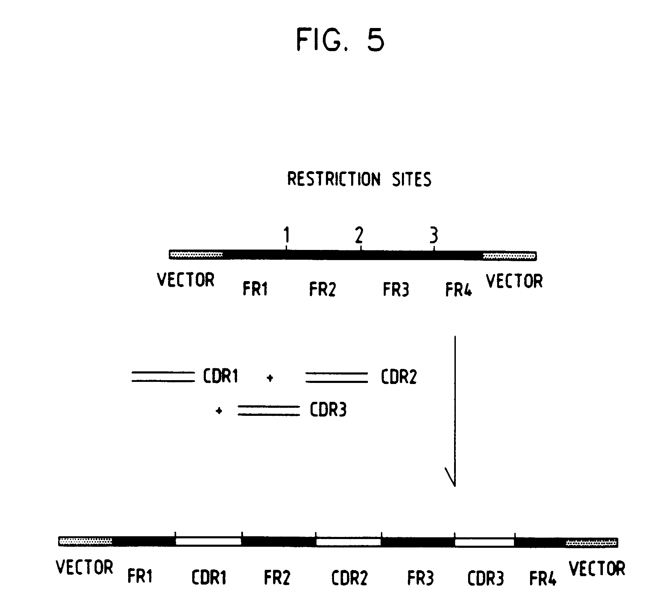

The present state of the art is such that the skilled man will be able to synthetize the DNA molecules of the invention given the information provided herein i.e. the amino acid sequences of the hypervariable regions and the DNA sequences coding for them. A method for constructing a variable domain gene is for example described in EPA 239 400 and may be briefly summarized as follows: A gene encoding a variable domain of a MAb of whatever specificity is cloned. The DNA segments encoding the framework and hypervariable regions are determined and the DNA segments encoding the hypervariable regions are removed so that the DNA segments encoding the framework regions are fused together with suitable restriction sites at the junctions. Double stranded synthetic CDR cassettes are prepared by DNA synthesis according to the sequences given in Seq. Id. No. 1; 2; 3 or 4. These cassettes are provided with sticky ends so that they can be ligated at the junctions of the framework. A protocol for achieving a DNA molecule encoding an immunoglobulin variable domain is shown in Figure 5.

Furthermore, it is not necessary to have access to the mRNA from a producing hybridoma cell line in order to obtain a DNA construct coding for the MAbs of the invention. Thus PCT application WO 90/07861 gives full instructions for the production of a MAb by recombinant DNA techniques given only written information as to the nucleotide sequence of the gene. The method comprises the synthesis of a number of oligonucleotides, their amplification by the PCR method, and their splicing to give the desired DNA sequence.

Expression vectors comprising a suitable promoter and genes encoding heavy and light chain constant parts are publicly available. Thus, once a DNA molecule of the invention is prepared it may be conveniently trans- ferred in an appropriate expression vector. DNA molecules encoding single chain antibodies may also be prepared by standard methods, for example, as described in WO 88/1649.

In view of the foregoing and since the mouse MAb as naturally secreted by the hybridoma is not the preferred type of MAb, it is considered that, although no deposit has been made of the hybridoma producing WN1 222-5 or WN1 58-9, nevertheless the present application discloses the invention in a manner sufficiently clear and complete for it to be carried out by a person skilled in the art.

In a particular embodiment of the invention, the recombinant means for the production of a LPS binding molecule includes first and second DNA constructs as described below:

The first DNA construct encodes a heavy chain or fragment thereof and comprises

a) a first part which encodes a variable domain comprising alternately framework and hypervariable regions, said hypervariable regions being in sequence hCDR1, hCDR2 and hCDR3, the amino acid sequences of which are shown in Seq. Id. No. 1 or 2; this first part starting with a codon encoding the first amino acid of the variable domain and ending with a codon encoding the last amino acid of the variable domain, and

b) a second part encoding a heavy chain constant part or fragment thereof which starts with a codon encoding the first amino acid of the constant part of the heavy chain and ends with a codon encoding the last amino acid of the constant part or fragment thereof, followed by a non-sense codon.

Preferably, this first part encodes a variable domain having an amino acid sequence substantially identical to the amino acid sequence as shown in Seq. Id. No. 1 or 2 starting with the amino acid at position 1 and ending with the amino acid at position 120. More preferably the first part has the nucleotide sequence as shown in Seq. Id. No. 1 or 2 starting with the nucleotide at position 1 and ending with the nucleotide at position 361. Also preferably, the second part encodes the constant part of a human heavy chain, more preferably the constant part of the human $\gamma$1 chain. This second part may be a DNA fragment of genomic origin (comprising introns) or a cDNA fragment (without introns). The sequence of Sequence Identifier 1 is more preferred than the sequence of Sequence Identifier No. 2

The second DNA construct encodes a light chain or fragment thereof and comprises

a) a first part which encodes a variable domain comprising alternately framework and hypervariable regions; said hypervariable regions being in sequence lCDR1, lCDR2 and lCDR3, the amino acid sequences of which are shown in Seq. Id. No. 3 or 4; this first part starting with a codon encoding the first amino acid of the variable domain and ending with a codon encoding the last amino acid of the variable domain, and

b) a second part encoding a light chain constant part or fragment thereof which starts with a coaon encoding the first amino acid of the constant part of the light chain and ends with a codon encoding the last amino acid of the constant part or fragment thereof followed by a non-sense codon.

Preferably, this first part encodes a variable domain having an amino acid sequence substantially identical to the amino acid sequence as shown in Seq. Id. No. 3 or 4 starting with the amino acid at position 1 and ending with the amino acid at position 107. More preferably, the first part has the nucleotide sequence as shown in Seq. Id. No. 3 or 4 starting with the nucleotide at position 1 and ending with the nucleotide at position 336. Also preferably the second part encodes the constant part of a human light chain, more preferably the constant part of the human κ chain.

In the first and second DNA constructs, the first and second parts are preferably separated by an intron. In the intron located between the first and second part, an enhancer is preferably inserted. The presence of this genetic element, which is transcribed but not translated, may be required for an efficient transcription of the second part. More preferably the first and second DNA constructs comprise the enhancer of a heavy chain gene.

The first or second DNA construct advantageously comprises a third part which is located upstream of the first part and which encodes a leader peptide. This peptide is required for secretion of the chains by the host organism in which they are expressed and is subsequently removed by the host organism. Preferably, the third part of the first DNA construct encodes a leader peptide of a heavy chain. Also preferably, the third part of the second DNA construct encodes a leader peptide of a light chain. Suitable leader peptides are indicated in Kabat et al. (supra). The structure of genes encoding the heavy and light chain of an Ig molecule is shown diagramatically in Figure 3A.

Each of the DNA constructs are placed under the control of suitable control sequences, in particular under the control of a suitable promoter. Any kind of promoter may be used, provided that it is adapted to the host organism in which the DNA constructs will be transferred for expression. However, if expression is to take place in a mammalian cell, it is particularly preferred to use the promoter of an immunoglobulin gene.

The desired antibody may be produced in a cell culture or in a transgenic animal. A suitable transgenic animal may be obtained according to standard methods which include microinjecting the first and second DNA constructs, placed under suitable control sequences, into fertilized ova, transferring the so prepared ova into appropriate pseudo-pregnant females and selecting a descendant expressing the desired antibody.

When the antibody chains are to be produced in a cell culture, the DNA constructs are advantageously inserted together or separately in an expression vector, the latter possibility being preferred. More preferably, they are separately inserted on two different but mutually compatible expression vectors.

Accordingly, the invention also provides an expression vector able to replicate in a prokaryotic or eukaryotic cell line which comprises at least one of the DNA constructs above described.

The next stage is the transfer of the expression vector or vectors containing the DNA constructs into a suitable host organism. When the DNA constructs are separately inserted on two expression vectors, they may be transferred separately, i.e. one type of vector per cell, or co- transferred, this latter possibility being preferred. A suitable host organism may be a bacteria, a yeast or a mammalian cell line, the last of these being preferred. More preferably, the mammalian cell line is of lymphoid origin e.g. a myeloma, hybridoma or a normal immortalized B-cell, but does not express any endogeneous antibody heavy or light chain.

It is also preferred that the host organism contains a large number of copies of the vectors per cell. If the host organism is a mammalian cell line, this desirable goal may be reached by amplifying the number of copies according to standard methods. Amplification methods usually consist of selecting for increased resistance to an antibiotic, said resistance being encoded by the expression vector.

In another aspect of the invention, there is provided a process for producing a multi-chain LPS binding molecule which comprises (i) culturing an organism which has been transformed with the first and second DNA constructs of the invention and (ii) recovering an active LPS binding molecule from the culture.

Alternatively, the heavy and light chains may be separately recovered and reconstituted into an active binding molecule after in vitro refolding. Reconstitution methods are well-known in the art; Examples of methods are in particular provided in EPA 120 674 or in EPA 125 023.

Therefore a process may also comprise

(i) culturing a first organism which is transformed with a first DNA construct of the invention and recovering said heavy chain or fragment thereof from the culture and

(ii) culturing a second organism which is transformed with a second DNA construct of the invention and recovering said light chain or fragment thereof from the culture and

(iii) reconstituting in vitro an active LPS binding molecule from the heavy chain or fragment thereof obtained in (i) and the light chain or fragment thereof obtained in (ii).

In a similar manner, there is also provided a process for producing a single chain or single domain LPS binding molecule which comprises (i) culturing an organism which is transformed with a DNA construct respectively encoding a single chain or single domain LPS binding molecule of the invention and (ii) recovering said molecule from the culture.

In the processes of the invention, it is most preferred that the DNA constructs are inserted into expression vectors.

LPS binding molecules of the invention exhibit very good protective activity against LPS of Gram-negative endotoxemia as shown both in the <u>in vitro</u> IL-6 assay described above, and in the following <u>in vivo</u> bioassay.

<u>Rabbit pyrogen model</u>

Rabbits are weighed and placed in restraining boxes. Probes from the APT 75 (Automatic Pyrogen Test Processor) are inserted in the rectum of each rabbit. The temperature of each rabbit is monitored every 15 minutes from 5 minutes after probe insertion, for an initial period of 95 minutes to establish a base/initial temperature (the base is the mean of the last three readings; if these show a greater than 0.3° range of fluctuation the test is not initialised).

Rabbits are then injected in a marginal ear vein with the LPS-binding molecule followed 30 min to 2 hr later by LPS in the same ear vein. LPS from different E. coli and salmonella, e.g. Salmonella abortus equi may be used. The suitable dose of LPS-binding molecule is to be determined, depending upon the type of molecule. For example WN1 222-5 is administered at 1 mg to 5 mg per kg body weight. For injection, this antibody is also prepared at 1 mg/ml in pyrogen-free saline and the LPS is injected at 10-100 ng/kg body weight, depending on the LPS used.

Control animals receive either LPS alone or the antibody alone. Rabbits are monitored at 15 min. intervals for a period starting from the injection and not exceeding 300 min.

The percentage of inhibition is measured as follows:

$$\% \text{ inhibition} = 100 - \frac{(\Delta T \text{ for Ab and LPS}) - (\Delta T \text{ for Ab alone})}{(\Delta T \text{ for LPS alone})} \times 100$$

$\Delta T$ = Temperature rise

In this assay, LPS binding molecules of the invention significantly reduce the increase of temperature in comparison with the negative control (LPS alone). Depending upon the type of LPS, the % of inhibition may reach levels well above 50%. A protective MAb may be defined in terms of this <u>in vivo</u> assay as one which gives at least 30% inhibition of fever 240 min after an LPS challenge of 10-100 ng/kg with an antibody dose of 1-5 mg/kg.

Therefore the invention also provides

(i) the use of an LPS binding molecule of the invention for preventing or treating gram-negative endotoxemia in humans

(ii) a method of preventing or treating gram-negative endotoxemia in humans which comprises administering an effective amount of an LPS binding molecule of the invention to a patient in need of such treatment.

(iii) a pharmaceutical composition for preventing or treating Gram-negative bacterial infections in humans which comprises an LPS binding molecule of the invention and a pharmaceutically acceptable carrier or diluent.

For these indications, the appropriate dosage will, of course, vary depending upon, for example, the particular molecule of the invention to be employed, the host, the mode of administration and the nature and severity of the condition being treated. However, in therapeutic use, satisfactory results are generally indicated to be obtained by administering at repeated intervals e.g. every two days or twice a week doses of from about 0.1 mg to about 15 mg per kilogram body weight as long as the patient is at risk. A molecule of the invention is conveniently administered parenterally, normally intravenously, for example, into the anticubital or other peripheral vein. A prophylactic treatment typically comprises administering a single dose of a molecule of the invention at a dosage of from about 20 µg to about 5 mg per Kg body weight.

Pharmaceutical compositions of the invention may be manufactured in conventional manner. A composition according to the invention is preferably provided in lyophilized form. For immediate administration it is dissolved in a suitable aqueous carrier, for example sterile water for injection or sterile buffered physiological saline. If it is considered desirable to make up a solution of larger volume for administration by infusion rather as a bolus injection, it is advantageous to incorporate human serum albumin or the patient's own heparinised blood or other sugar stabilisers into the saline at the time of formulation. The presence of an excess of such physiologically inert protein prevents loss of monoclonal antibody by adsorption onto the walls of the container and tubing used with the infusion solution. If albumin is used, a suitable concentration is from 0.5 to 4.5% by weight of the saline solution.

LPS binding molecules of the invention, either unlabelled or, preferably, labelled with a radioactive isotope or a fluorescent marker, may also be used for diagnostic purposes to determine the nature, location and extent of Gram-negative bacterial infections, or analytically to detect the presence of LPS or Gram-negative bacterial contamination in water, foodstuffs, biological fluids, etc. Thus for example a labelled LPS binding molecule of the invention may be useful for the imaging of localised infectious foci for surgical removal or other treatment. The LPS binding molecules of the invention may also be attached to a solid phase support-material to form the solid phase of an affinity chromatography purification system for the removal of LPS molecules from biological fluids, e.g. blood serum.

Brief description of the drawings

Figure 1 shows the detailed structure of a Salmonella LPS molecule, indicating the various Ra-Re rough mutant types. In this Figure, Abe = abequose, Ac = acetyl, Ara = 4-amino-4-deoxy-L-arabinose, Etn = ethanolamine, FA = hydroxy fatty acid, Gal = D-galactose, Glc = D-glucose, GlcN = D-glucosamine, GlcNAc = N-acetyl-d-glucosamine, Hep = heptose, KD0 = 2-keto-3-deoxyoctonic acid, Man = mannose, P = phosphate, Rha = L-rhamnose. Dotted lines indicate incomplete substitution.

Figure 2 is a representation of the outer core structures R1; R2; R3; R4 and K12 of different E. coli strains. The same abbreviations are used as in Fig. 1.

Figure 3A is a schematic diagram showing the structure of an IgG molecule as well as the genes encoding heavy and light chains respectively designated (1) and (2). Figure 3B schematically represents the arrangement of a variable domain of a heavy or light chain into framework (FR) and hypervariable (CDR) regions.

Figure 4A shows the binding capacity of monoclonal antibody WN1 222-5 against different LPS molecules derived from eight different E. coli strains as determined by Western blotting. The drawing represents the spots of the gel. The strains are described in detail in Tables II & III and the lane numbering represents: St = standard; 1 = E. coli 0111B4; 2 = E. coli 086; 3 = E. coli 018K-; 4 = E. coli 016; 5 = E. coli 015; 6 = E. coli 012; 7 = E. coli 06; and 8 = E. coli 04.

Figure 4B shows the binding capacity of WN1 58-9 against different LPS molecules derived from eight different bacterial strains as determined by Western blotting. The drawing represents the spots of the gel. The lane numbering represents: St = standard; 1 = S. minnesota wild type; 2 = E. coli 018; 3 = E. coli 016; 4 = E. coli 015; 5 = E. coli 012; 6 = E. coli 06; 7 = E. coli 04; and 8 = E. coli 02.

Figure 4C shows the binding capacity of H1 61-2 against different LPS molecules derived from eight different bacterial strains as determined by Western blotting. The drawing represents the spots of the gel. The lane numbering represents: St standard; 1 = S. minnesota wild type; 2 = E. coli 018K-; 3 = E. coli 04; 4 = E. coli 06; 5 = E. coli 012; 6 = E. coli 015; 7 = E. coli 016; and 8 = E. coli 086.

Figure 4D shows the binding capacity of SZ27 19.16.07 against different LPS molecules derived from eight different bacterial strains as determined by Western blotting. The drawing represents the spots of the gel. The lane numbering represents: St = standard; 1 = E. coli 04; 2 = = E. coli 016; 3 = E. coli 018K-; 4 = K235; 5 = $R_1$B; 6 = $R_2$B; 7 = $R_3$B and 8 = $R_4$B.

Figure 5 shows a protocol for constructing CDR replacements by insertion of CDR cassettes into a vector containing 4 framework regions fused together.

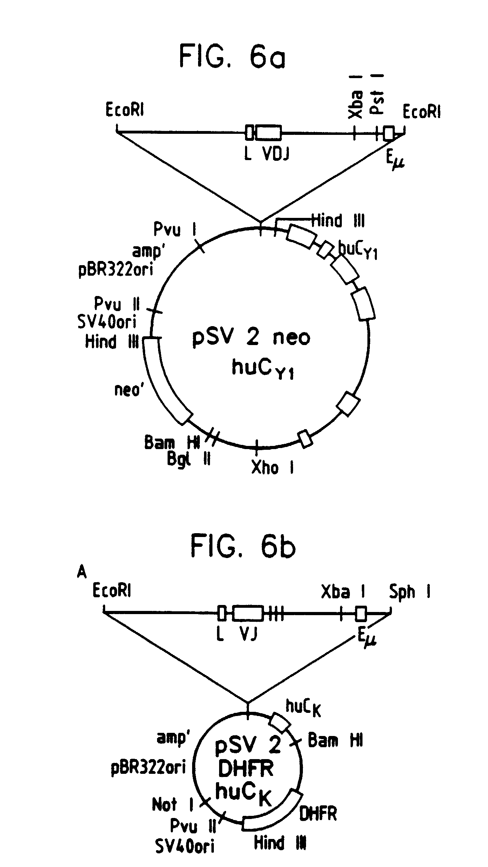

Figures 6A and 6B show the parental expression vectors pSV-2 neo and pSV-2DHFR. Both plasmids comprise an ampicillin resistance gene ($amp^R$) and the origin of replication of pBR322 and SV40 (pBR322 ori and SV40 or). pSV-2neo is characterized by the presence of a neomycin gene ($neo^R$) and the gene encoding the human $\gamma_1$ constant part (hu C$\gamma_1$) while pSV-2 DHFR has inserted a dihydrofolate reductase (DHFR) gene (methotrexate resistance) and the gene encoding the human κ constant part (hu C$_\kappa$). The final vectors for expressing the chimeric heavy or light chain are respectively obtained by inserting into pSV-2neo a DNA fragment encoding the leader peptide (L), and the variable domain (VDJ4) of the WN1 222-5 heavy chain together with the mouse heavy chain enhancer and by inserting into pSV2-DHFR a DNA fragment encoding the leader peptide (L) and the variable domain (VJ$_1$) of the WN1 222-5 light chain together with the mouse heavy chain enhancer.

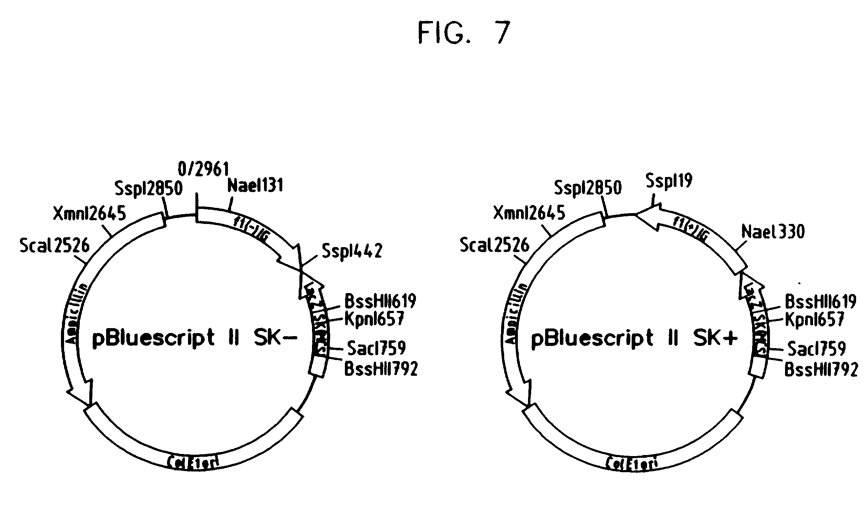

Figure 7 shows a drawing of the cloning vector p Bluescript II SK- and p Bluescript SK+ (Stratagene).

The following Examples illustrate the invention:

Example 1: Preparation of Murine Monoclonal Antibody WN1 222-5

a) Immunization Procedure

New Zealand Black mice were immunized i.v. with $10^8$ heat-killed baceria in 0.1 ml. Four injections were carried out, as follows:

week 1    EcR2 + EcR3 + SmR60
week 2    EcR1 + EcR4 + Ec018 rough strain

week 3    EcR2 + EcR3 + SmR60
week 4    EcR1 + EcR4 + Ec018 rough strain

Antibody responses were monitored in tail bleed samples, and a mouse was selected for boosting on the basis of its strong plural response profile.

After one month, two injections, one day apart, of a cocktail of the 6 different strains ($10^8$ heat-killed bacteria) were given, the first injection i.v., the second i.p.

b) Fusion

On the fourth day after boosting, spleen cells were recovered and fused with the non-secreting murine B cell lymphoma PAI-0 cell line, using standard procedures. Supernatant from wells containing growing hybridomas were screened using cocktails of different smooth and rough LPS as described above, and hybridomas producing cross-reactive MAb's were cloned.

One of the resulting clones was WN1 222-5, which secretes a murine MAb of the IgG2ak isotype. The WN1 222-5 MAb was purified from culture supernatants collected after in-vitro fermentation of the WN1 222-5 clone and made pyrogen-free by treatment with detergent.

The reactivity pattern of WN1 222-5 is shown in Tables II A and III A.

Example 2: Preparation of Murine Monoclonal Antibody WN1 58-9

WN1 58-9 is a further clone obtained from the fusion described in Example 1. Its reactivity pattern is shown in Tables II B and III B.

Example 3: Preparation of Murine Monoclonal Antibody H1 61-2

Balb/c mice were immunized i.v. with a cocktail of EcK12, EcR2 and EcR3 ($10^8$ heat-killed bacteria, four injections one week apart). Antibody responses were monitored in tail bleed samples, and a mouse was selected for boosting on the basis of its strong plural response profile.

After one month, two injections, one day apart, of a cocktail of the three different strains ($10^8$ heat-killed bacteria) were given. The first injection was i.v., the second i.p. On the fourth day the spleen cells were fused with the PAI-O cell line using standard procedures.

Primary screening was carried out using the following cocktails of different rough LPS:

    1) complete core: EcR2, EcR3, EcK12
    2) complete core: EcR1, EcR4, SmR60
    3) Rb2        : SmR345
    4) Rc         : EcJ4, St878, SmR5
    5) Rd         : SmR7, SmR4
    6) Re         : EcK12, StSL1102, StSL1181, SmR595
    7) Lipid A      : EcK12, SmR595
    8) Negative Control: BSA

Hybridomas producing cross-reactive MAb's were cloned, and one of the resulting clones was H1 61-2, which secretes a murine MAb of the IgGlk isotype. The H1 61-2 MAb was purified from culture supernatants, and gave the reactivity pattern shown in Tables IIC and III C.

Example 4: Preparation of Murine Monoclonal Antibody SZ 27 19.16.07

Balb/c female mice were immunized with $10^8$ heat-killed bacteria in 0.1 ml i.v. on each of six days (day 0, 1, 2, 7, 8 and 9). Different immunogens were used, at 28 day intervals between starting each immunogen. The immunogens used were

| 1st 6 injections | P. aeruginosa PAC-605 |
|---|---|
| 2nd "        " | EcR1 |
| 3rd "        " | EcR2 |
| 4th "        " | EcR3 |

A group of 5 mice received identical immunizations. Antibody responses were monitored in tail bleed samples to purified LPS antigen from the following strains:

S. typh. Ra*, Rb*, Rc*, Rd and Re
S. Minnesota Lipid A
E. coli R1*, C61*, K12, Re (strain D31m4) and Lipid A (ex D31m4)
P. aeruginosa C605*

Strong antibody responses to the marked antigens * had developed after cyclic immunization with 4 different bacteria, and a mouse was selected for boosting on the basis of its strong plural response profile.

Six weeks after completion of the last series of immunization, the selected mouse was boosted i.v. with a cocktail of $2.10^8$ heat-killed bacteria of each of E. coli R1, R2, R3, R4 and K12. The spleen was removed three days later for fusion.

Fusion was carried out with the NS-0 cell line using standard procedures.

Primary screening was carried out using two LPS cocktails:

1) S. minnesota Ra + Rc + Re
2) E. coli C62 + K12 + Re

260 hybridoma supernatants were screened, and 20 of those showing strong responses to both cocktails were selected for further growth. These were then given a secondary screening on 11 different LPS antigens before selection for cloning. These were:

S. typh. Ra, Rb, Rc, Rd and Re
S. minn. Lipid A
E. coli R1, K12, Re, Lipid A
P. aerug. C605.

A number of hybridomas, including SZ27 19 showed the following reaction patterns:

| strong | S. typh Ra; E. coli R1 |
|---|---|
| weak | S. typh Rb, Rc; P. aerug. 602 |
| negative | S. typh Rd, Re; E. coli lipid A, K12, Re |

After subcloning, the clone SZ27 19.16.07 was isolated. It produced a murine MAb of the IgG2ak isotype.

The reaction pattern of this antibody is shown in Tables II D and III D.

Example 5: Cloning of the WN1 222-5 or WN1 58-9 heavy chain variable region by Polymerase Chain Reaction (PCR) and construction of a chimeric gene

Cloning step No. 1

The amino terminal sequence of the heavy chain is determined as being Glu-Val-Lys-Leu-Val-Glu-Ser-Gly. Based on this an upstream primer complementary to the mRNA encoding the end of the expected leader sequence and the amino acid sequence cited above is constructed so that its nucleotide sequence reads:

```
              Sal 1

5' AGGT GTC GAC TCC GAG GTG AAG CTG GTG GAG TCT GG 3'

             Glu Val Lys Leu Val Glu Ser Gly
```

A downstream primer complementary to the mRNA encoding a fragment of the mouse γ2a constant part is also constructed so that its nucleotide sequence reads 5' TCCAGGTCAAGGTCACTG 3'.

The upstream and downstream primers are used together to amplify a DNA fragment encoding the variable region of the WN1 222-5 heavy chain from a WN1 222-5 mRNA preparation. The amplified DNA fragment is then sequenced and its V, D and J segments are determined.

Cloning Step No. 2

Another downstream primer complementary to the J segment and BstEll
having the nucleotide sequence 5' GGAGACGGTGACCGAGGTT 3' is constructed.

To introduce the BstEll restriction site, the original DNA-sequence as naturally found, is slightly modified.

The J-specific downstream primer and the upstream primer already used in the cloning step No. 1 are used to amplify a DNA fragment encoding the variable region of the WN1 222-5 heavy chain from a WN1 222-5 cDNA preparation. The amplification of the WN1 58-9 is made analogously to the one of WN1 222-5. The amplified DNA fragment is further cleaved with Sal I and BstE II and cloned into a heavy chain cassette treated with the same enzyme.

The heavy chain cassette is prepared as follows:
A 2.3 kb EcoRI-SalI DNA fragment comprising the promoter and the leader sequence of the gene encoding the heavy chain of the RFT2 antibody (Heinrich et al, J. of Immunol. (1989) 143: 3589) is cloned into the polylinker region of the cloning vector pBluescript II SK-(Stratagene). Downstream from this insertion a 0.4 kb BstEll-BamHI DNA fragment comprising the J segment and the beginning of the major intron of the gene encoding an anti-cytomegalovirus antibody (Newkirk et al, J. Clin. Invest. (1988) 81: 1511).

The EcoRI-BamHI fragment is then transferred into pSV2-neo-Eμ-huCγ1 (Heinrich et al; supra) which contains the human heavy chain enhancer (Eμ) and the sequence encoding the human γl constant part.

Example 6 Cloning of the WN1-222-5 or WN1 58-9 Light Chain Variable Region by PCR and Construction of a Chimeric Gene

Cloning steps No. 1 and 2 of Example 5 are repeated using the following primers:
Upstream primer:

```
                    MluI

        5'  AGGT ACG CGT TGT GAC ATC CAG ATG AAC CAG TCT CC 3'

            Thr Arg Cys Val Ile Gln Met Asn Gln Ser Pro
```

Downstream primer specific for the κ constant part:

```
            5'  GCACACGACTGAGGCCACCTC 3'
```

Downstream primer specific for the J segment

```
                        HindIII

            5'  CGTTTGATTTCAAGCTTGGTG 3'
```

The amplified DNA fragment is further cleaved with MluI and HindIII and cloned into a light chain cassette treated with the same enzyme. WN1 222-5 and WN1 58-9 are analogously treated.

The light chain cassette is prepared as follows: A 1.3 kb EcoRI-MluI DNA fragment comprising the promoter and the leader sequence of the gene encoding the light chain of the RFT2 antibody (Heinrich et al; Supra) is cloned into the polylinker region of the cloning vector pBluescript II SK- (Stratagene). Downstream from this insertion, a 0.4 kb HindIII-Xbal DNA fragment comprising the J segment and the beginning of the major intron of the gene encoding light chain of RFT2 is cloned.

The EcoRI-Xbal fragment is then transferred into pSV2-DHFR-Eμ-huCκ which is constructed as follows:

A 1.1 kb Xbal - Xbal fragment encoding the murine heavy chain enhancer (Heinrich et al; supra) together with a SphI - HindIII fragment encoding the human κ constant part is subcloned in phage M13 mp18 (Boehringer Mannheim). After disruption of restriction sites by mutagenesis a filled-in EcoRI - HindIII fragment comprising the sequence for the murine heavy chain enhancer (Eμ) and the human κ constant part (huCκ) is cloned in the filled in EcoRI - BamHI site of pSV2-DHFR.

Example 7 Expression of a WN1 222-5 or WN1 58-9 chimeric antibody

The expression vectors as obtained in Examples 5 and 6 are co-transferred in a mouse myeloma cell line SP2/0 (ATCC CRL 1581) by electroporation using a gene pulser apparatus from Bio Rad Laboratories. This technique is known to create stable transfectants at a high frequency. The SP2/0 cell line fails to produce endogeneous heavy and light chains and is sensitive to Gentamycin (G 418) at a concentration of 0.8 mg/l.

SP2/0 cells are grown in the usual growth medium (RPMI + 10% FCS + $5 \times 10^{-5}$ β-mercaptoethanol) harvested in the log phase of growth and washed with the electroporation buffer (Bio-Rad). Cell concentration is adjusted to $2 \times 10^{7}$ cells/ml. To 0.8 ml of the cell suspension is added 15-20 µg of each plasmid. The mixture is placed on ice and left to stand for 10 min. Then the cells are subjected to an electrical pulse (280 Volt; 25 µF) and again left to stand for 15 min. Cells are transferred to the usual growth medium and incubated at 37°C in a $CO_2$ incubator.

After 3-day incubation, selection for G 418 resistance is started. Cells are resuspended in fresh medium containing 1.4 mg/ml G 418. The cultures yield growing cells after 10-14 day-incubation in the presence of G 418. After 2-week incubation, the supernatants of the confluent cultures are tested for human IgG expression in a sandwich-type ELISA (anti-human κ-light chain / supernatant /anti-human IgG-alkaline phosphatase conjugate).

This test indicates that complete antibody molecules are secreted in all cultures at varying concentrations in the range of 50-500 ng/ml.

To select cells in which the DHFR gene is amplified and therefore secrete high amounts of the desired antibody two selection procedures for Methotrexate (MTX) resistance are carried out as described below. For this purpose, the G 418 resistant cell pools are each divided and amplification is proceeded either according to procedure A (MTX increase by a factor of 2 or 2.5) or procedure B (MTX increase by a factor of 5).

```
         G418-resistant Cells      G418-resistant Cells


                 Procedure A        Procedure B
                      |                  |
                100nM MTX          200nM MTX
                      |                  |
                250nM MTX            1µM MTX
                      |                  |
                500nM MTX            5µM MTX
                      |                  |
                  1µM MTX           25µM MTX
                      |                  |
                2.5µM MTX          100µM MTX
                      |
                  5µM MTX
                      |
                 10µM MTX
                      |
                 25µM MTX
                      |
                100µM MTX
```

Each amplification step comprises inoculating the cells at a density of $2x10^5$ cells/ml in the usual growth medium supplemented with G 418 at 1.4 mg/ml and with MTX at the concentration of choice. After 72 hour incubation, cells and the supernatant separated. Antibody secretion is monitored either by ELISA or by HPLC using a protein A column.

Most of the pools reach a maximum of specific antibody production at a certain MTX concentration. The best producing pools are cloned by limiting dilution. Subsequently, the antibody is purified from a culture supernatant by elution on a protein A affinity column.

SEQUENCE IDENTIFIER No.1

**Subject matter:**     The immunoglobulin heavy chain variable domain of the WN1 222-5 antibody

**Sequence type:**      Nucleotide sequence and its corresponding amino acid sequence

**Length:**     361 nucleotides

**Original source:**    A murine hybridoma

| Features of the amino acid sequence: | | |
|---|---|---|
| hFR1 | from a.a. | 1 to 30 |
| hCDR1 | from a.a | 31 to 35 |
| hFR2 | from a.a. | 36 to 49 |
| hCDR2 | from a.a. | 50 to 67 |
| hFR3 | from a.a. | 68 to 100 |
| hCDR3 | from a.a. | 101 to 109 |
| hFR4 | from a.a. | 110 to 120. |

```
GAG GTG AAG CTG GTG GAG TCT GGA GGA GGC TTG GTA CAG CCG GGG GGT      48
Glu Val Lys Leu Val Glu Ser Gly Gly Gly Leu Val Gln Pro Gly Gly
              5                   10                  15

TCT CTG AGT CTC TCC TGT GCA GCT TCT GGA TTC ACC TTC AGT GAT TAC      96
Ser Leu Ser Leu Ser Cys Ala Ala Ser Gly Phe Thr Phe Ser Asp Tyr
              20                  25                  30

TAC ATG ACC TGG GTC CGC CAG GCT CCA GGG AAG GCA CCT GAG TGG TTG     144
Tyr Met Thr Trp Val Arg Gln Ala Pro Gly Lys Ala Pro Glu Trp Leu
              35                  40                  45

GCT TTG ATT AGA AAC AAA CGT AAT GGT GAC ACA GCA GAG TAT AGT GCA     192
Ala Leu Ile Arg Asn Lys Arg Asn Gly Asp Thr Ala Glu Tyr Ser Ala
         50                  55                  60

TCT GTG AAG GGT CGG TTC ACC ATC TCC AGA GAT TAT TCC CGA AGC ATC     240
Ser Val Lys Gly Arg Phe Thr Ile Ser Arg Asp Tyr Ser Arg Ser Ile
    65                  70                  75                  80

CTC CAT CTT CAA ATG AAT GCC CTG AGA ACT GAG GAC AGT GCC ACT TAT     288
Leu His Leu Gln Met Asn Ala Leu Arg Thr Glu Asp Ser Ala Thr Tyr
              85                  90                  95

TAT TGT GTA AGA CAG GGA CGG GGC TAT ACT TTG GAC TAT TGG GGT CAA     336
Tyr Cys Val Arg Gln Gly Arg Gly Tyr Thr Leu Asp Tyr Trp Gly Gln
         100                 105                 110

GGA ACC TCA GTC ACC GTC TCC TCA G                                   361
Gly Thr Ser Val Thr Val Ser Ser
         115                 120
```

SEQUENCE IDENTIFIER No.2

**Subject matter:**     The immunoglobulin heavy chain variable domain of the WN1 58-9 antibody

**Sequence type:**     Nucleotide sequence and its corresponding amino acid sequence

**Length:**     361 nucleotides

**Original source:**     A murine hybridoma

| Features of the amino acid sequence: | | |
|---|---|---|
| hFR1ᵣ | from a.a. | 1 to 30 |
| hCDR1 | from a.a. | 31 to 35 |
| hFR2ᵣ | from a.a. | 36 to 49 |
| hCDR2 | from a.a. | 50 to 67 |
| hFR3ᵣ | from a.a. | 68 to 100 |
| hCDR3 | from a.a. | 101 to 109 |
| hFR4 | from a.a. | 110 to 120. |

```
GAG GTG AAG CTG GTG GAG TCT GGA GGA GGC TTG GTA CAG CCT GGG GGT      48
Glu Val Lys Leu Val Glu Ser Gly Gly Gly Leu Val Gln Pro Gly Gly
                 5                   10                  15

TCT CTG CGT CTC TCC TGT GCA GCT TCT GGA TTC ACC TTC ATT GAT TAC      96
Ser Leu Arg Leu Ser Cys Ala Ala Ser Gly Phe Thr Phe Ile Asp Tyr
             20                  25                  30

TAC ATG ACC TGG GTC CGC CAT CCG CCA GGG GAG GCA CCT GAA TGG TTG     144
Tyr Met Thr Trp Val Arg His Pro Pro Gly Glu Ala Pro Glu Trp Leu
         35                  40                  45

GCT TTG ATT AGA AAC TAC CGT AAT GGT GAC ACA GCA GAA TAC AGT GCA     192
Ala Leu Ile Arg Asn Tyr Arg Asn Gly Asp Thr Ala Glu Tyr Ser Ala
     50                  55                  60

TCT GTG AGG GGT CGG TTC ACC ATC TCC AGA GAT GAT TCC CAA AGC ATC     240
Ser Val Arg Gly Arg Phe Thr Ile Ser Arg Asp Asp Ser Gln Ser Ile
 65                  70                  75                  80

CTC TAT CTT CAA ATG AAT GCC CTG AGA GCT GAG GAC AGT GCC ACT TAT     288
Leu Tyr Leu Gln Met Asn Ala Leu Arg Ala Glu Asp Ser Ala Thr Tyr
                 85                  90                  95

TAC TGT GTA AGA CAG GGA CGG GGC TAT ACT CTG GAC TAC TGG GGT CAA     336
Tyr Cys Val Arg Gln Gly Arg Gly Tyr Thr Leu Asp Tyr Trp Gly Gln
             100                 105                 110

GGA ACC TCA GTC ACC GTC TCC TCA                                     360
Gly Thr Ser Val Thr Val Ser Ser
         115                 120
```

SEQUENCE IDENTIFIER No.3

**Subject matter:** The immunoglobulin light chain variable domain of the WN1 222-5 antibody

**Sequence type:** Nucleotide sequence and its corresponding amino acid sequence

**Length:** 321 nucleotides

**Original source:** A murine hybridoma

19

| Features of the amino acid sequence: | | |
|---|---|---|
| IFR1 | from a.a. | 1 to 23 |
| ICDR1 | from a.a. | 24 to 34 |
| IFR2 | from a.a. | 35 to 49 |
| ICDR2 | from a.a. | 50 to 56 |
| IFR3 | from a.a. | 57 to 88 |
| ICDR3 | from a.a. | 89 to 97 |
| IFR4 | from a.a. | 98 to 107. |

DNA and AA sequence
Light Chain:

```
GAC ATC CAG ATG AAC CAG TCT CCA TCC AGT CTG TCT GCA TCC CTC          45
Asp Ile Gln Met Asn Gln Ser Pro Ser Ser Leu Ser Ala Ser Leu
            5                   10                  15

GGA GAC ACA ATT TCC ATC ACT TGC CGT GCC AGT CAG AAC ATT AAT          90
Gly Asp Thr Ile Ser Ile Thr Cys Arg Ala Ser Gln Asn Ile Asn
            20              25                  30

ATT TGG TTA AGC TGG TAT CAG CAA AAA CCA GGA AAT GTT CCT AAA         135
Ile Trp Leu Ser Trp Tyr Gln Gln Lys Pro Gly Asn Val Pro Lys
            35                  40                  45

CTT TTA ATC TAT AAG GCT TCC AAC TTG CAC ACA GGC GTC CCA TCA         180
Leu Leu Ile Tyr Lys Ala Ser Asn Leu His Thr Gly Val Pro Ser
            50                  55                  60

AGG TTT AGT GGC AGT GGA TCT GGA ACA GAT TTC ACA TTA ATC ATC         225
Arg Phe Ser Gly Ser Gly Ser Gly Thr Asp Phe Thr Leu Ile Ile
            65                  70                  75

AGC AGT CTG CAG CCT GAA GAC ATT GCC ACT TAC TAC TGT CTA CAG         270
Ser Ser Leu Gln Pro Glu Asp Ile Ala Thr Tyr Tyr Cys Leu Gln
            80                  85                  90

GGT CAA AGT TAT CCT CGT ACG TTC GGT GGA GGC ACC AAG CTG GAG         315
Gly Gln Ser Tyr Pro Arg Thr Phe Gly Gly Gly Thr Lys Leu Glu
            95                  100                 105

ATC AAA C                                                           322
Ile Lys
107
```

SEQUENCE IDENTIFIER No.4

**Subject matter:**      The immunoglobulin light chain variable domain of the WN1 58-9 antibody

**Sequence type:**      Nucleotide sequence and its corresponding amino acid sequence

**Length:**      361 nucleotides

**Original source:**      A murine hybridoma

| Features of the amino acid sequence: | | |
|---|---|---|
| IFR1$_r$ | from a.a. | 1 to 23 |
| ICDR1 | from a.a. | 24 to 34 |
| IFR2$_r$ | from a.a. | 35 to 49 |
| ICDR2 | from a.a. | 50 to 56 |
| IFR3$_r$ | from a.a. | 57 to 88 |
| ICDR3 | from a.a. | 89 to 97 |
| IFR4 | from a.a. | 98 to 107. |

```
GAC ATC CAG ATG AAC CAG TCT CCA TCC AGT CTG TCT GCA TCC CTC        45
Asp Ile Gln Met Asn Gln Ser Pro Ser Ser Leu Ser Ala Ser Leu
            5                   10                  15

GGA GAC ACA ATT ACC ATC ACT TGC CGT GCC AGA CTG AAC ATT AAT        90
Gly Asp Thr Ile Thr Ile Thr Cys Arg Ala Arg Leu Asn Ile Asn
            20                  25                  30

ATT TGG TTA AGT TGG TAC CAG CAG AAA GCA GGA AAT ATT CCT AAA        135
Ile Trp Leu Ser Trp Tyr Gln Gln Lys Ala Gly Asn Ile Pro Lys
            35                  40                  45

CTT TTG ATC TCT AAG GCT TCC AAC TTG CAC ACA GGC GTC CCA TCA        180
Leu Leu Ile Ser Lys Ala Ser Asn Leu His Thr Gly Val Pro Ser
            50                  55                  60

AGG TTT AGT GGC AGT GGA TCT GGA ACA GAT TTC ACA TTA ACC ATC        225
Arg Phe Ser Gly Ser Gly Ser Gly Thr Asp Phe Thr Leu Thr Ile
            65                  70                  75

AGC AGT CTG CGG CCT GAA GAC ATT GCC ACT TAC TAC TGT CTA CAG        270
Ser Ser Leu Arg Pro Glu Asp Ile Ala Thr Tyr Tyr Cys Leu Gln
            80                  85                  90

GGT CAA AGT TAT CCT CGT ACG TTC GGT GGA GGC ACC AAG CTT GAA        315
Gly Gln Ser Tyr Pro Arg Thr Phe Gly Gly Gly Thr Lys Leu Glu
            95                  100                 105

ATC AAA                                                            321
Ile Lys
107
```

Table I

| Region | Location on the heavy chains | Location on the light chains |
|---|---|---|
| FR1/FR1$_r$ | amino acid 1 to 30 | amino acid 1 to 23 |
| CDR1/CDR1 | amino acid 31 to 35 | amino acid 24 to 34 |
| FR2/FR2$_r$ | amino acid 36 to 49 | amino acid 35 to 49 |
| CDR2/CDR2 | amino acid 50 to 67 | amino acid 50 to 56 |
| FR3/FR3$_r$ | amino acid 68 to 100 | amino acid 57 to 88 |
| CDR3 | amino acid 101 to 109 | amino acid 89 to 97 |
| FR4 | amino acid 110 to 120 | amino acid 98 to 107 |

# T A B L E  II A  (first page)

| CHEMOTYPE mAb | | STRAIN | | SUPPLIER | WN1 222-5 | | |
|---|---|---|---|---|---|---|---|
| | | | | | 100 ng/ml | 10 ng/ml | 1 ng/ml |
| LPS | Smooth | E. coli | 02 | Univ. Edinburgh | +++++ | ++++ | ++ |
| LPS | Smooth | E. coli | 04 | Univ. Edinburgh | +++++ | +++++ | ++++ |
| LPS | Smooth | E. coli | 06 | Univ. Edinburgh | +++++ | +++++ | +++ |
| LPS | Smooth | E. coli | 012 | Univ. Edinburgh | +++++ | +++++ | ++ |
| LPS | Smooth | E. coli | 015 | Univ. Edinburgh | +++++ | +++++ | ++++ |
| LPS | Smooth | E. coli | 016 | Univ. Edinburgh | +++++ | +++++ | ++ |
| LPS | Smooth | E. coli | 018K- | Univ. Edinburgh | +++++ | +++++ | +++ |
| LPS | Smooth | E. coli | 018K+ | Univ. Edinburgh | +++++ | +++++ | ++++ |
| LPS | Smooth | E. coli | 026B6 | Difco | +++++ | +++++ | +++ |
| LPS | Smooth | E. coli | 055B5 | Difco | +++++ | ++ | + |
| LPS | Smooth | E. coli | 075 | Univ. Edinburgh | +++++ | +++++ | +++ |
| LPS | Smooth | E. coli | 086 | Univ. Edinburgh | +++++ | +++++ | +++ |
| LPS | Smooth | E. coli . | 0111B4 | Difco | +++++ | +++++ | ++ |
| LPS | Smooth | E. coli | 0127B8 | Difco | +++++ | +++++ | +++ |
| LPS | Smooth | E. coli | 0128B12 | Difco | ++++ | +++ | ++ |
| LPS | Smooth | E. coli | K235 | List | +++++ | +++++ | +++ |
| | | | | | | | |
| LPS | Smooth | S. minnesota | wt | List | +++++ | +++++ | +++ |
| LPS | Smooth | S. typhimurium | wt | Difco | +++++ | +++++ | ++ |
| LPS | Smooth | S. typhimurium | BO ag 0:4,5,12 (SH 4809) | Bio-Carb | +++++ | ++++ | + |
| LPS | Smooth | S. typhimurium | BO ag 0:1,4,5,12 (SL 3622) | Bio-Carb | +++++ | ++++ | + |
| LPS | Smooth | S. typhimurium | BO ag 0:4,5,12^2 (SH 4305) | Bio-Carb | ++++++ | +++++ | + |
| LPS | Smooth | S. typhi | DO ag 0:9,12^2 (253 Ty) | Bio-Carb | +++++ | +++++ | +++ |
| LPS | Smooth | S. newport | C2 ag 0:6,8 | Bio-Carb | +++++ | +++++ | +++ |
| LPS | Smooth | S. enteridis | DO ag 0:9,12 (SH 1262) | Bio-Carb | +++++ | +++++ | +++ |
| LPS | Smooth | S. thompson | C1 ag 0:6,7, (1s40) | Bio-Carb | +++++ | +++++ | ++++ |
| LPS | Smooth | S. abortus equi | (H1178) | Institut Borstel | +++++ | +++++ | ++++ |

EP 0 576 439 B1

# T A B L E  II A (second page)

| | | | | | | | |
|---|---|---|---|---|---|---|---|
| LPS | cCore | E. coli | K12 | Univ. Edinburgh | +++++ | +++++ | ++ |
| LPS | cCore | E. coli | C62 | Univ. Edinburgh | ++++ | +++++ | ++ |
| LPS | cCore | E. coli | R1 | Institut Borstel | +++++ | +++++ | ++ |
| LPS | cCore | E. coli | R2 | Institut Borstel | +++++ | +++++ | ++ |
| LPS | cCore | E. coli | R3 | Institut Borstel | +++++ | +++++ | ++++ |
| LPS | cCore | E. coli | R4 | Institut Borstel | +++++ | +++++ | +++ |
| LPS | cCore | S. minnesota | Ra R60 | List | +++++ | +++++ | +++ |
| LPS | cCore | S. typhimurium | TV119 | Sigma | +++++ | +++++ | +++ |
| LPS | cCore | S. typhimurium | 1542 | Univ. Edinburgh | +++++ | +++++ | ++++ |
| LPS | cCore | K. aerogenes | M10B | Univ. Edinburgh | − | − | − |
| | | | | | | | |
| LPS | Rb2 | S. minnesota | R345 | List | ++++++ | +++++ | ++++ |
| LPS | Rb3 | S. minnesota | | Bio-Carb | ++++++ | +++++ | ++ |
| | | | | | | | |
| LPS | Rc | E. coli | J5 | List | ++++++ | +++++ | ++++ |
| LPS | Rc | S. typhimurium | 878 | Univ. Edinburgh | ++++++ | +++++ | ++ |
| LPS | Rc | S. typhimurium | SL684 | Sigma | ++++++ | +++++ | ++++ |
| LPS | Rc | P. aeruginosa | PAC605 | Univ. Edinburgh | + | + | + |
| | | | | | | | |
| LPS | RcP− | S. minnesota | R5 | List | ++++++ | +++++ | ++++ |
| | | | | | | | |
| LPS | Rd2 | E. coli | F583 | Sigma | ++++++ | +++++ | +++ |
| LPS | Rd1P− | S. minnesota | R7 | List | +++ | ++ | + |
| LPS | Rd2 | S. minnesota | R4 (V594) | Institut Borstel | ++++ | +++ | ++ |
| | | | | | | | |
| LPS | Re | E. coli | K12 (D31m4) | List | ++++ | ++++ | ++ |
| LPS | Re | E. coli | F515 | Institut Borstel | +++++ | +++++ | ++++ |
| LPS | Re | S. minnesota | R595 | List | + | + | − |
| LPS | Re | S. typhimurium | SL1102 | Univ. Edinburgh | − | − | − |
| LPS | Re | S. typhimurium | SL1181 | Sigma | − | − | − |

EP 0 576 439 B1

# T A B L E  II A    (third page)

| Lipid A | E. coli | K12 (ex-D31m4) | List | – | – | – |
|---------|---------|----------------|------|---|---|---|
| Lipid A | S. minnesota | R595 | List | – | – | – |
| BSA | | | | – | – | – |

Purified native LPS (2 ug/ml) were used to coat the plates
Values are reported as O.D., one + equals 0.5 O.D. (405 nm).

EP 0 576 439 B1

EP 0 576 439 B1

# T A B L E  II B  (first page)

| CHEMOTYPE | | STRAIN | | SUPPLIER | WN1 58-9 MAb | | |
| --- | --- | --- | --- | --- | --- | --- | --- |
| | | | | | 100 ng/ml | 10ng/ml | 1 ng/ml |
| LPS | Smooth | E. coli | 02 | Univ. Edinburgh | ++++ | ++++ | + |
| LPS | Smooth | E. coli | 04 | Univ. Edinburgh | +++++ | ++++ | + |
| LPS | Smooth | E. coli | 06 | Univ. Edinburgh | ++++ | +++++ | − |
| LPS | Smooth | E. coli | 012 | Univ. Edinburgh | ++++ | +++++ | + |
| LPS | Smooth | E. coli | 015 | Univ. Edinburgh | +++++ | +++++ | ++ |
| LPS | Smooth | E. coli | 016 | Univ. Edinburgh | ++++ | +++++ | + |
| LPS | Smooth | E. coli | 018K− | Univ. Edinburgh | ++++ | ++++ | + |
| LPS | Smooth | E. coli | 018K+ | Univ. Edinburgh | +++++ | +++++ | ++ |
| LPS | Smooth | E. coli | 026B6 | Difco | +++++ | +++++ | + |
| LPS | Smooth | E. coli | 055B5 | Difco | +++ | + | − |
| LPS | Smooth | E. coli | 075 | Univ. Edinburgh | ++++ | ++ | − |
| LPS | Smooth | E. coli | 086 | Univ. Edinburgh | ++++ | ++++ | + |
| LPS | Smooth | E. coli | 0111B4 | Difco | ++++ | ++++ | + |
| LPS | Smooth | E. coli | 0127B8 | Difco | ++++ | +++ | + |
| LPS | Smooth | E. coli | 0128B12 | Difco | ++ | ++ | − |
| LPS | Smooth | E. coli | K235 | List | ++++ | ++++ | + |
| | | | | | | | |
| LPS | Smooth | S. minnesota | wt | List | ++++ | ++++ | + |
| LPS | Smooth | S. typhimurium | wt | Difco | ++++ | ++++ | + |
| LPS | Smooth | S. typhimurium | BO ag 0:4,5,12 (SH 4809) | Bio-Carb | ++++ | +++ | − |
| LPS | Smooth | S. typhimurium | BO ag 0:1,4,5,12 (SL 3622) | Bio-Carb | ++++ | +++ | + |
| LPS | Smooth | S. typhimurium | BO ag 0:4,5,12^2 (SH 4305) | Bio-Carb | ++++ | ++++ | + |
| LPS | Smooth | S. typhi | DO ag 0:9, 12^2 (253 Ty) | Bio-Carb | +++++ | +++++ | + |
| LPS | Smooth | S. newport | C2 ag 0:6,8 | Bio-Carb | +++++ | +++++ | + |
| LPS | Smooth | S. enteridis | DO ag 0:9,12 (SH 1262) | Bio-Carb | +++++ | ++++ | + |
| LPS | Smooth | S. thompson | C1 ag 0:6,7, (1s40) | Bio-Carb | +++++ | +++++ | ++ |
| LPS | Smooth | S. abortus equi | (H1178) | Institut Borstel | +++++ | +++++ | ++ |
| LPS | cCore | E. coli | K12 | Univ. Edinburgh | ++++ | ++++ | + |
| LPS | cCore | E. coli | C62 | Univ. Edinburgh | +++++ | +++++ | +++ |
| LPS | cCore | E. coli | R1 | Institut Borstel | +++++ | +++++ | + |
| LPS | cCore | E. coli | R2 | Institut Borstel | ++++ | +++ | + |
| LPS | cCore | E. coli | R3 | Institut Borstel | +++++ | +++++ | + |

## T A B L E  II B  (second page)

| | | | | | | | | |
|---|---|---|---|---|---|---|---|---|
| LPS | cCore | E. coli | | R4 | | Institut Borstel | ++++ | +++++ | + |
| LPS | cCore | | S. minnesota | Ra R60 | | List | ++++ | +++++ | + |
| LPS | cCore | | S. typhimurium | TV119 | | Sigma | ++++ | ++++ | + |
| LPS | cCore | | S. typhimurium | 1542 | | Univ. Edinburgh | ++++ | +++++ | + |
| LPS | cCore | | K. aerogenes | M10B | | Univ. Edinburgh | - | - | - |
| LPS | Rb2 | | S. minnesota | R345 | | List | +++++ | +++++ | +++ |
| LPS | Rb3 | | S. minnesota | | | Bio-Carb | +++++ | +++++ | + |
| LPS | Rc | E. coli | | J5 | | List | +++++ | +++++ | + |
| LPS | Rc | | S. typhimurium | 878 | | Univ. Edinburgh | +++++ | +++++ | + |
| LPS | Rc | | P. typhimurium | SL684 | | Sigma | +++++ | +++++ | ++ |
| LPS | Rc | | P. aeruginosa | PAC605 | | Univ. Edinburgh | + | + | - |
| LPS | RcP⁻ | | S. minnesota | R5 | | List | +++++ | +++++ | ++ |
| LPS | Rd2 | E. coli | | F583 | | Sigma | +++++ | +++++ | ++ |
| LPS | Rd1P⁻ | | S. minnesota | R7 | | List | ++++ | ++ | - |
| LPS | Rd2 | | S. minnesota | R4 (V594) | | Institut Borstel | ++++ | +++ | - |
| LPS | Re | E. coli | | K12 (D31m4) | | List | +++++ | +++++ | ++ |
| LPS | Re | E. coli | | F515 | | Institut Borstel | +++++ | +++++ | +++ |
| LPS | Re | | S. minnesota | R595 | | List | + | - | - |
| LPS | Re | | S. typhimurium | SL1102 | | Univ. Edinburgh | - | - | - |
| LPS | Re | | S. typhimurium | SL1181 | | Sigma | - | - | - |
| Lipid A | | E. coli | | K12 (ex-D31m4) | | List | - | - | - |
| Lipid A | | | S. minnesota | R595 | | List | - | - | - |
| BSA | | | | | | | - | - | - |

Purfied native LPS (2 µg/ml) were used to coat the plates
Values are reported as O.D., one + equals 0.5 O.D. (405 nm).
BSA = bovine serum albumin

EP 0 576 439 B1

# T A B L E   II C   (first page)

| CHEMOTYPE | | | STRAIN | SUPPLIER | H1 61-2 IgG1 | | | |
|---|---|---|---|---|---|---|---|---|
| | | | | | 1µg/ml | 100 ng/ml | 10ng/ml | 1 ng/ml |
| LPS | Smooth | E. coli | 02 | Univ. Edinburgh | ++++ | +++++ | ++ | − |
| LPS | Smooth | E. coli | 06 | Univ. Edinburgh | +++ | +++++ | ++ | − |
| LPS | Smooth | E. coli | 012 | Univ. Edinburgh | +++++ | +++++ | +++++ | +++ |
| LPS | Smooth | E. coli | 015 | Univ. Edinburgh | +++++ | +++++ | ++++ | + |
| LPS | Smooth | E. coli | 016 | Univ. Edinburgh | + | ++ | − | − |
| LPS | Smooth | E. coli | 018K− | Univ. Edinburgh | +++++ | +++++ | +++++ | + |
| LPS | Smooth | E. coli | 018K+ | Univ. Edinburgh | +++++ | +++++ | +++++ | ++ |
| LPS | Smooth | E. coli | 026B6 | Difco | +++++ | +++++ | +++++ | + |
| LPS | Smooth | E. coli | 055B5 | Difco | +++ | ++++ | − | − |
| LPS | Smooth | E. coli | 075 | Univ. Edinburgh | +++ | +++++ | +++ | − |
| LPS | Smooth | E. coli | 086 | Univ. Edinburgh | +++++ | +++++ | ++++ | + |
| LPS | Smooth | E. coli | 0111B4 | Difco | +++++ | +++++ | +++++ | ++ |
| LPS | Smooth | E. coli | 0127B8 | Difco | +++++ | +++++ | +++++ | +++ |
| LPS | Smooth | E. coli | 0128B12 | Difco | − | − | − | − |
| LPS | Smooth | E. coli | K235 | List | ++++ | +++++ | +++ | − |
| LPS | Smooth | S. minnesota | wt | List | ++++ | ++++ | ++ | − |
| LPS | Smooth | S. typhimurium | wt | Difco | +++++ | +++++ | +++++ | + |
| LPS | cCore | E. coli | K12 | Univ. Edinburgh | +++++ | +++++ | +++++ | +++ |
| LPS | cCore | E. coli | C62 | Univ. Edinburgh | ++++++ | +++++ | +++++ | ++ |
| LPS | cCore | E. coli | R1 | Institut Borstel | ++++++ | +++++ | +++++ | +++ |
| LPS | cCore | E. coli | R2 | Institut Borstel | +++++ | +++++ | +++++ | ++++ |
| LPS | cCore | E. coli | R3 | Institut Borstel | +++++ | +++++ | +++++ | ++ |
| LPS | cCore | E. coli | R4 | Institut Borstel | ++++++ | +++++ | +++++ | ++++ |

EP 0 576 439 B1

# T A B L E  II C  (second page)

| | | | | | | | | |
|---|---|---|---|---|---|---|---|---|
| LPS | cCore | S. minnesota | Ra R60 | List | ++++++ | +++++ | +++++ | ++++ |
| LPS | cCore | S. typhimurium | TV119 | Sigma | ++++++ | +++++ | +++++ | + |
| LPS | cCore | S. typhimurium | 1542 | Univ. Edinburgh | +++++ | +++++ | +++++ | ++ |
| LPS | cCore | K. aerogenes | M10B | Univ. Edinburgh | – | – | – | – |
| LPS | Rb2 | S. minnesota | R345 | List | ++++++ | +++++ | ++ | – |
| LPS | Rc | E. coli | J5 | List | ++++++ | +++++ | +++++ | ++++ |
| LPS | Rc | S. typhimurium | 878 | Univ. Edinburgh | ++++++ | ++++++ | +++++ | ++++ |
| LPS | Rc | P. typhimurium | SL684 | Sigma | +++++ | +++++ | +++++ | ++++ |
| LPS | Rc | P. aeruginosa | PAC605 | Univ. Edinburgh | – | – | – | – |
| LPS | RcP⁻ | S. minnesota | R5 | List | ++++++ | +++++ | +++++ | +++ |
| LPS | Rd2 | E. coli | F583 | Sigma | +++ | ++ | – | – |
| LPS | Rd1P⁻ | S. minnesota | R7 | List | – | – | – | – |
| LPS | Rd2 | S. minnesota | R4 | Institut Borstel | – | – | – | – |
| LPS | Re | E. coli | K12 (D31m4) | List | – | – | – | – |
| LPS | Re | E. coli | F515 | Institut Borstel | – | – | – | – |
| LPS | Re | S. minnesota | R595 | List | – | – | – | – |
| LPS | Re | S. typhimurium | SL1102 | Univ. Edinburgh | – | – | – | – |
| LPS | Re | S. typhimurium | SL1181 | Sigma | – | – | – | – |
| Lipid A | | E. coli | K12 (ex-D31m4) | | – | – | – | – |
| Lipid A | | S. minnesota | R595 | | + | ++ | – | – |
| BSA | | | | | – | – | – | – |

Purfied native LPS (2 µg/ml) were used to coat the plates
Values are reported as O.D., one + equals 0.5 O.D. (405 nm).
BSA = bovine serum albumin

EP 0 576 439 B1

## T A B L E  II D (first page)

| CHEMOTYPE | | | STRAIN | SUPPLIER | SZ 27 19.16.7 | | | |
|-----------|---|---|--------|----------|-------|----------|---------|---------|
| | | | | | 1µg/ml | 100 ng/ml | 10ng/ml | 1 ng/ml |
| LPS | Smooth | E. coli | 02 | Univ. Edinburgh | ++ | + | − | − |
| LPS | Smooth | E. coli | 06 | Univ. Edinburgh | − | − | − | − |
| LPS | Smooth | E. coli | 012 | Univ. Edinburgh | +++++ | ++++++ | ++++ | + |
| LPS | Smooth | E. coli | 015 | Univ. Edinburgh | + | +++ | ++ | − |
| LPS | Smooth | E. coli | 016 | Univ. Edinburgh | − | − | − | − |
| LPS | Smooth | E. coli | 018K− | Univ. Edinburgh | + | +++ | − | − |
| LPS | Smooth | E. coli | 018K+ | Univ. Edinburgh | ++++ | ++++ | ++ | − |
| LPS | Smooth | E. coli | 026B6 | Difco | +++++ | +++++ | +++ | − |
| LPS | Smooth | E. coli | 055B5 | Difco | − | − | − | − |
| LPS | Smooth | E. coli | 075 | Univ. Edinburgh | + | + | − | − |
| LPS | Smooth | E. coli | 086 | Univ. Edinburgh | ++ | ++++ | +++ | − |
| LPS | Smooth | E. coli | 0111B4 | Difco | +++++ | +++++ | +++++ | + |
| LPS | Smooth | E. coli | 0127B8 | Difco | ++++++ | +++++ | +++++ | + |
| LPS | Smooth | E. coli | 0128B12 | Difco | − | − | − | − |
| LPS | Smooth | E. coli | K235 | List | +++ | ++ | ++ | − |
| LPS | Smooth | S. minnesota | wt | List | ++ | − | − | − |
| LPS | Smooth | S. typhimurium | wt | Difco | +++++ | +++++ | +++ | − |
| LPS | cCore | E. coli | K12 | Univ. Edinburgh | +++++ | +++++ | +++++ | + |
| LPS | cCore | E. coli | C62 | Univ. Edinburgh | +++++ | +++++ | +++++ | + |
| LPS | cCore | E. coli | R1 | Institut Borstel | +++++ | +++++ | +++++ | + |
| LPS | cCore | E. coli | R2 | Institut Borstel | ++++++ | +++++ | +++++ | + |
| LPS | cCore | E. coli | R3 | Institut Borstel | +++++ | +++++ | +++++ | + |
| LPS | cCore | E. coli | R4 | Institut Borstel | +++++ | +++++ | +++++ | + |

EP 0 576 439 B1

# T A B L E   II D   (second page)

| LPS | cCore | S. minnesota | Ra R60 | List | +++++ | +++++ | +++++ | + |
|-----|-------|--------------|--------|------|-------|-------|-------|---|
| LPS | cCore | S. typhimurium | TV119 | Sigma | +++++ | +++++ | ++ | − |
| LPS | cCore | S. typhimurium | 1542 | Univ. Edinburgh | +++++ | +++++ | ++++ | + |
| LPS | cCore | K. aerogenes | M10B | Univ. Edinburgh | − | − | − | − |
| LPS | Rb2 | S. minnesota | R345 | List | +++++ | ++++ | ++ | − |
| LPS | Rc | E. coli | J5 | List | +++++ | +++++ | +++++ | + |
| LPS | Rc | S. typhimurium | 878 | Univ. Edinburgh | +++++ | +++++ | +++++ | + |
| LPS | Rc | P. typhimurium | SL684 | Sigma | ++++++ | +++++ | +++++ | + |
| LPS | Rc | P. aeruginosa | PAC605 | Univ. Edinburgh | + | − | − | − |
| LPS | RcP⁻ | S. minnesota | R5 | List | +++++ | +++++ | ++++ | + |
| LPS | Rd2 | E. coli | F583 | Sigma | − | − | − | − |
| LPS | Rd1P⁻ | S. minnesota | R7 | List | − | − | − | − |
| LPS | Rd2 | S. minnesota | R4 | Institut Borstel | − | − | + | + |
| LPS | Re | E. coli | K12 (D31m4) List | | − | − | − | − |
| LPS | Re | E. coli | F515 | Institut Borstel | − | − | − | − |
| LPS | Re | S. minnesota | R595 | List | − | − | − | − |
| LPS | Re | S. typhimurium | SL1102 | Univ. Edinburgh | − | − | − | − |
| LPS | Re | S. typhimurium | SL1181 | Sigma | − | − | − | − |
| Lipid A | | E. coli | K12 (ex-D31m4) List | | − | − | − | − |
| Lipid A | | S. minnesota | R595 | List | − | − | − | − |
| BSA | | | | | − | − | − | − |

Purfied native LPS (2 µg/ml) were used to coat the plates
Values are reported as O.D., one + equals 0.5 O.D. (405 nm).
BSA = bovine serum albumin

EP 0 576 439 B1

# T A B L E   III A   (first page)

| CHEMOTYPE | | STRAIN | SUPPLIER | WN1 222-5 |
|---|---|---|---|---|
| | | | | 100 ng/ml |
| Bacteria smooth | E. coli | 01 | Univ. Edinburgh | ++++ |
| Bacteria smooth | E. coli | 04 | Univ. Edinburgh | ++++ |
| Bacteria smooth | E. coli | 06 | Univ. Edinburgh | ++++ |
| Bacteria smooth | E. coli | 015 | Univ. Edinburgh | ++++ |
| Bacteria smooth | E. coli | 016 | Univ. Edinburgh | ++++ |
| Bacteria smooth | E. coli | 018K- | Univ. Edinburgh | ++++ |
| Bacteria smooth | E. coli | 075 | Univ. Edinburgh | |
| Bacteria cCore | E. coli | K12 | Univ. Edinburgh | +++++ |
| Bacteria cCore | E. coli | C62 | Univ. Edinburgh | +++++ |
| Bacteria cCore | E. coli | R1 | Institut Borstel | +++++ |
| Bacteria cCore | E. coli | R2 | Institut Borstel | +++++ |
| Bacteria cCore | E. coli | R3 | Institut Borstel | +++++ |
| Bacteria cCore | E. coli | R4 | Institut Borstel | +++++ |
| Bacteria cCore | S. minnesota | Ra R60 | Univ. Edinburgh | +++++ |
| Bacteria cCore | S. typhimurium | 1135 | Univ. Edinburgh | +++++ |
| Bacteria cCore | S. typhimurium | 1542 | Univ. Edinburgh | +++++ |
| Bacteria cCore | K. aerogenes | M10B | Univ. Edinburgh | − |
| Bacteria Rb | S. minnesota | R345 | Univ. Edinburgh | ++++ |
| Bacteria Rc | E. coli | J5 | Univ. Edinburgh | +++++ |
| Bacteria Rc | S. typhimurium | 878 | Univ. Edinburgh | +++++ |
| Bacteria Rc | P. aeruginosa | PAC 605 | Univ. Edinburgh | − |
| Bacteria Rc | S. minnesota | R5 | Univ. Edinburgh | ++++ |
| Bacteria Rd1P- | S. minnesota | R7 | Univ. Edinburgh | ++++ |
| Bacteria Rd2 | S. minnesota | R4 | Univ. Edinburgh | − |

EP 0 576 439 B1

# T A B L E   III A   (second page)

| | | | | |
|---|---|---|---|---|
| Bacteria Re | E. coli | F515 | Institut Borstel | - |
| Bacteria Re | S. minnesota | R595 | Univ. Edinburgh | - |
| Bacteria Re | S. typhimurium | SL1102 | Univ. Edinburgh | + |
| BSA | | | | - |

Heat killed bacteria (0.5 x $10^8$ cell/ml) were used to coat the plates
Values are reported as O.D., one + equals 0.5 O.D. (405 nm).

EP 0 576 439 B1

EP 0 576 439 B1

# T A B L E   III B   (first page)

| CHEMOTYPE | | STRAIN | SUPPLIER | WN1 58-9 100 ng/ml |
|---|---|---|---|---|
| Bacteria smooth | E. coli | 01 | Univ. Edinburgh | +++++ |
| Bacteria smooth | E. coli | 04 | Univ. Edinburgh | +++++ |
| Bacteria smooth | E. coli | 06 | Univ. Edinburgh | +++++ |
| Bacteria smooth | E. coli | 015 | Univ. Edinburgh | +++++ |
| Bacteria smooth | E. coli | 016 | Univ. Edinburgh | +++++ |
| Bacteria smooth | E. coli | 018K- | Univ. Edinburgh | +++++ |
| Bacteria smooth | E. coli | 075 | Univ. Edinburgh | +++++ |
| | | | | |
| Bacteria cCore | E. coli | K12 | Univ. Edinburgh | +++++ |
| Bacteria cCore | E. coli | C62 | Univ. Edinburgh | +++++ |
| Bacteria cCore | E. coli | R1 | Institut Borstel | ++++ |
| Bacteria cCore | E. coli | R2 | Institut Borstel | ++++ |
| Bacteria cCore | E. coli | R3 | Institut Borstel | +++++ |
| Bacteria cCore | E. coli | R4 | Institut Borstel | ++++ |
| Bacteria cCore | S. minnesota | Ra R60 | Univ. Edinburgh | +++++ |
| Bacteria cCore | S. typhimurium | 1135 | Univ. Edinburgh | +++++ |
| Bacteria cCore | S. typhimurium | 1542 | Univ. Edinburgh | +++++ |
| Bacteria cCore | K. aerogenes | M10B | Univ. Edinburgh | - |
| | | | | |
| Bacteria Rb | S. minnesota | R345 | Univ. Edinburgh | ++++ |
| | | | | |
| Bacteria Rc | E. coli | J5 | Univ. Edinburgh | +++++ |
| Bacteria Rc | S. typhimurium | 878 | Univ. Edinburgh | +++++ |
| Bacteria Rc | P. aeruginosa | PAC 605 | Univ. Edinburgh | - |
| | | | | |
| Bacteria Rc | S. minnesota | R5 | Univ. Edinburgh | +++++ |
| | | | | |
| Bacteria Rd1P- | S. minnesota | R7 | Univ. Edinburgh | +++++ |
| Bacteria Rd2 | S. minnesota | R4 | Univ. Edinburgh | - |

# T A B L E   III B   (second page)

| Bacteria Re | E. coli | F515 | Institut Borstel | – |
| Bacteria Re | S. minnesota | R595 | Univ. Edinburgh | – |
| Bacteria Re | S. typhimurium | SL1102 | Univ. Edinburgh | + |
| BSA | | | | – |

Heat killed bacteria ($0.5 \times 10^8$ cell/ml) were used to coat the plates
Values are reported as O.D., one + equals 0.5 O.D. (405 nm).

EP 0 576 439 B1

# T A B L E III C (first page)

| CHEMOTYPE | | STRAIN | SUPPLIER | H1 61-2 IgG1 | | | |
|---|---|---|---|---|---|---|---|
| | | | | 1µg/ml | 100 ng/ml | 10ng/ml | 1ng/nl |
| Bacteria smooth | E. coli | 01 | Univ. Edinburgh | +++++ | +++++ | +++++ | +++ |
| Bacteria smooth | E. coli | 04 | Univ. Edinburgh | +++++ | +++++ | +++++ | ++ |
| Bacteria smooth | E. coli | 06 | Univ. Edinburgh | +++++ | +++++ | +++++ | ++ |
| Bacteria smooth | E. coli | 015 | Univ. Edinburgh | +++++ | +++++ | +++++ | + |
| Bacteria smooth | E. coli | 016 | Univ. Edinburgh | +++++ | +++++ | +++++ | +++ |
| Bacteria smooth | E. coli | 018K- | Univ. Edinburgh | + | − | − | − |
| Bacteria smooth | E. coli | 075 | Univ. Edinburgh | +++++ | +++++ | +++++ | ++++ |
| Bacteria cCore | E. coli | K12 | Univ. Edinburgh | +++++ | +++++ | +++++ | +++ |
| Bacteria cCore | E. coli | C62 | Univ. Edinburgh | +++++ | +++++ | +++++ | ++++ |
| Bacteria cCore | E. coli | R1 | Institut Borstel | +++++ | +++++ | +++++ | +++ |
| Bacteria cCore | E. coli | R2 | Institut Borstel | +++++ | +++++ | +++++ | ++++ |
| Bacteria cCore | E. coli | R3 | Institut Borstel | +++++ | +++++ | +++++ | ++++ |
| Bacteria cCore | E. coli | R4 | Institut Borstel | +++++ | +++++ | +++++ | ++++ |
| Bacteria cCore | S. minnesota | Ra R60 | Univ. Edinburgh | ++++++ | +++++ | +++++ | +++ |
| Bacteria cCore | S. typhimurium | 1135 | Univ. Edinburgh | +++++ | +++++ | +++++ | ++++ |
| Bacteria cCore | S. typhimurium | 1542 | Univ. Edinburgh | +++++ | +++++ | +++++ | ++++ |
| Bacteria cCore | K. aerogenes | M10B | Univ. Edinburgh | − | − | − | − |
| Bacteria Rb | S. minnesota | R345 | Univ. Edinburgh | ++ | + | − | − |
| Bacteria Rc | E. coli | J5 | Univ. Edinburgh | +++++ | +++++ | +++++ | ++ |
| Bacteria Rc | S. typhimurium | 878 | Univ. Edinburgh | +++++ | +++++ | +++++ | +++ |
| Bacteria Rc | P. aeruginosa | PAC 605 | Univ. Edinburgh | − | − | − | − |
| Bacteria Rc | S. minnesota | R5 | Univ. Edinburgh | +++++ | +++++ | +++++ | ++ |
| Bacteria Rd1P- | S. minnesota | R7 | Univ. Edinburgh | +++++ | ++++ | ++ | + |
| Bacteria Rd2 | S. minnesota | R4 | Univ. Edinburgh | − | − | − | − |

EP 0 576 439 B1

# T A B L E  III C (second page)

| | | | | | | | | |
|---|---|---|---|---|---|---|---|---|
| Bacteria Re | E. coli | F515 | Institut Borstel | – | | – | – | – |
| Bacteria Re | S. minnesota | R595 | Univ. Edinburgh | – | | – | – | – |
| Bacteria Re | S. typhimurium | SL1102 | Univ. Edinburgh | – | | – | – | – |
| | | | | | | | | |
| BSA | | | | – | | – | – | – |

Heat killed bacteria (0.5 x $10^8$ cell/ml) were used to coat the plates
Values are reported as O.D., one + equals 0.5 O.D. (405 nm).

EP 0 576 439 B1

# T A B L E  III D  (first page)

| CHEMOTYPE | | STRAIN | SUPPLIER | SZ 27 19.16.7 | | | |
|---|---|---|---|---|---|---|---|
| | | | | 1µg/ml | 100 ng/ml | 10ng/ml | 1ng/nl |
| Bacteria smooth | E. coli | 01 | Univ. Edinburgh | ++++++ | +++++ | +++++ | ++ |
| Bacteria smooth | E. coli | 04 | Univ. Edinburgh | ++++++ | +++++ | +++++ | ++ |
| Bacteria smooth | E. coli | 06 | Univ. Edinburgh | +++++ | +++++ | +++++ | ++ |
| Bacteria smooth | E. coli | 015 | Univ. Edinburgh | ++++++ | +++++ | +++++ | + |
| Bacteria smooth | E. coli | 016 | Univ. Edinburgh | ++++++ | +++++ | +++++ | ++ |
| Bacteria smooth | E. coli | 018K- | Univ. Edinburgh | ++++ | +++ | + | − |
| Bacteria smooth | E. coli | 075 | Univ. Edinburgh | +++++ | +++ | +++++ | + |
| Bacteria cCore | E. coli | K12 | Univ. Edinburgh | +++++ | +++++ | +++++ | ++ |
| Bacteria cCore | E. coli | C62 | Univ. Edinburgh | +++++ | +++++ | +++++ | ++ |
| Bacteria cCore | E. coli | R1 | Institut Borstel | +++++ | +++++ | +++++ | + |
| Bacteria cCore | E. coli | R2 | Institut Borstel | +++++ | +++++ | +++++ | + |
| Bacteria cCore | E. coli | R3 | Institut Borstel | +++++ | +++++ | +++++ | + |
| Bacteria cCore | E. coli | R4 | Institut Borstel | +++++ | +++++ | +++++ | ++ |
| Bacteria cCore | S. minnesota | Ra R60 | Univ. Edinburgh | +++++ | +++++ | +++++ | + |
| Bacteria cCore | S. typhimurium | 1135 | Univ. Edinburgh | ++++++ | +++++ | +++++ | + |
| Bacteria cCore | S. typhimurium | 1542 | Univ. Edinburgh | ++++++ | +++++ | +++++ | ++ |
| Bacteria cCore | K. aerogenes | M10B | Univ. Edinburgh | − | − | − | − |
| Bacteria Rb | S. minnesota | R345 | Univ. Edinburgh | + | − | − | − |
| Bacteria Rc | E. coli | J5 | Univ. Edinburgh | ++++++ | +++++ | +++++ | + |
| Bacteria Rc | S. typhimurium | 878 | Univ. Edinburgh | ++++++ | +++++ | +++++ | + |
| Bacteria Rc | P. aeruginosa | PAC 605 | Univ. Edinburgh | − | − | − | − |
| Bacteria Rc | S. minnesota | R5 | Univ. Edinburgh | ++++++ | +++++ | +++++ | + |
| Bacteria Rd1P- | S. minnesota | R7 | Univ. Edinburgh | +++++ | ++++ | ++ | − |
| Bacteria Rd2 | S. minnesota | R4 | Univ. Edinburgh | − | − | − | − |

EP 0 576 439 B1

# T A B L E   III D   (second page)

| Bacteria Re | E. coli | F515 | Institut Borstel | – | | – | – | – |
| Bacteria Re | S. minnesota | R595 | Univ. Edinburgh | – | | – | – | – |
| Bacteria Re | S. typhimurium | SL1102 | Univ. Edinburgh | – | | – | – | – |
| BSA | | | | – | | – | – | – |

Heat killed bacteria ($0.5 \times 10^8$ cell/ml) were used to coat the plates
Values are reported as O.D., one + equals 0.5 O.D. (405 nm).

EP 0 576 439 B1

**Claims**

1.  A monoclonal antibody which recognizes an epitope in the core region of the LPS molecule and which is cross-protective against endotoxemia caused by at least two different Gram-negative bacterial strains having different core structures, said monoclonal antibody having a constant region of human origin, a variable framework region of human or nonhuman origin, and a hypervariable region of nonhuman origin.

2.  A monoclonal antibody according to Claim 1 which recognizes an epitope which is completely present in the Rc core structure of E. coli and is also present in the complete core.

3.  A monoclonal antibody according to Claim 1 or Claim 2 wherein the hypervariable region is of murine origin.

4.  A monoclonal antibody according to any one of the preceding claims which is of IgG isotype.

5.  A hybridoma cell line producing a monoclonal antibody according to any one of the preceding claims.

6.  An LPS binding molecule which comprises at least one antigen binding site comprising at least one domain which comprises in sequence, the hypervariable regions hCDR1, hCDR2 and hCDR3;

    said hCDR1 having the amino acid sequence Asp Tyr Tyr Met Thr;
    said hCDR2 having the amino acid sequence Leu Ile Arg Asn W Arg Asn Gly Asp Thr Ala Glu Tyr Ser Ala Ser Val X;
        wherein W is Lys or Tyr and X is Lys or Arg;
    said hCDR3 having the amino acid sequence Gln Gly Arg Gly Tyr Thr Leu Asp Tyr;

    and direct equivalents thereof.

7.  A single domain antibody according to Claim 6 comprising in sequence the hypervariable regions hCDRI, hCDR2 and hCDR3 associated with murine or human heavy chain framework regions so as to form an isolated heavy chain variable domain.

8.  An LPS binding molecule according to Claim 6 comprising at least one antigen binding site comprising;

    a) a first domain comprising in sequence the hypervariable regions hCDR1, hCDR2 and hCDR3 as defined in claim 6 and,
    b) a second domain comprising in sequence the hyper-variable regions lCDR1, lCDR2 and 1CDR3;

        said lCDRI having the amino acid sequence Arg Ala Y Z Asn Ile Asn Ile Trp Leu Ser,
        wherein Y is Ser or Arg and Z is Gln or Leu;
        said lCDR2 having the amino acid sequence Lys Ala Ser Asn Leu His Thr;
        said lCDR3 having the amino acid sequence Leu Gln Gly Gln Ser Tyr Pro Arg Thr;
        and direct equivalents thereof.

9.  An LPS binding molecule according to Claim 8 in which the hypervariable regions are associated with murine or human framework regions.

10. An LPS binding molecule according to Claim 8 or Claim 9 in which the first and the second domains are part of a single common peptide chain.

11. A single chain antibody according to Claim 10 in which the first and the second domains are respectively an Ig heavy chain variable domain and an Ig light chain variable domain, and are covalently bound by a peptide linker consisting of from 10 to 30 amino acids.

12. An LPS binding molecule according to Claim 8 or Claim 9 in which the first domain is part of a heavy chain of at least a fragment of an Ig molecule, and the second domain is part of a light chain of at least a fragment of an Ig molecule.

13. An LPS binding molecule according to Claim 12 which is a complete Ig molecule.

**14.** An Ig molecule according to Claim 13 which is of IgG isotype.

**15.** An Ig molecule according to Claim 13 or Claim 14 which is murine.

**16.** An Ig molecule according to Claim 13 or Claim 14 in which the variable domains are murine and the constant domains are human.

**17.** An Ig molecule according to Claim 13 or Claim 14 in which the framework regions and the constant domains are human.

**18.** An Ig molecule according to Claim 15 or Claim 16 in which the heavy chain variable domain has an amino acid sequence substantially identical to that given in Seq. Id. No 1 or alternatively in Seq. Id. No 2 and the light chain variable domain has an amino acid sequence substantially identical to that given in Seq. Id. No 3 or alternatively in Seq. Id. No 4.

**19.** An Ig molecule according to Claim 18 as dependent o n Claim 16, in which the heavy chain constant domain is of human type $\gamma_1$ and the light chain constant domain is of human type $\kappa$.

**20.** A DNA construct coding for an amino acid sequence comprising in sequence the hypervariable regions hCDR1, hCDR2 and hCDR3 stated in Claim 6.

**21.** A DNA construct encoding a heavy chain or fragment thereof and comprising

a) a first part which encodes a variable domain comprising alternately framework and hypervariable regions, said hypervariable regions being in sequence hCDR1, hCDR2 and hCDR3, the amino acid sequences of which are stated in Claim 6; this first part starting with a codon encoding the first amino acid of the variable domain and ending with a codon encoding the last amino acid of the variable domain, and
b) a second part encoding a heavy chain constant part or fragment thereof which starts with a codon encoding the first amino acid of the constant part of the heavy chain and ends with a codon encoding the last amino acid of the constant part or fragment thereof, followed by a non-sense codon.

**22.** A DNA construct according to Claim 21 in which the first part encodes a variable domain having an amino acid sequence substantially identical to the amino acid sequence as shown in Seq. Id. No 1 or alternatively No. 2 and the second part encodes the constant part of the human $\gamma_1$ chain.

**23.** A DNA construct coding for an amino acid sequence comprising in sequence the hypervariable regions lCDR1, lCDR2 and lCDR3 stated in Claim 8.

**24.** A DNA construct encoding a light chain or fragment thereof and comprising

a) a first part which encodes a variable domain comprising alternately framework and hypervariable regions; said hypervariable regions being in sequence lCDR1, lCDR2 and lCDR3, the amino acid sequences of which are shown in Seq. Id. No. 3 or in Seq. Id. No 4; this first part starting with a codon encoding the first amino acid of the variable domain and ending with a codon encoding the last amino acid of the variable domain, and
b) a second part encoding a light chain constant part or fragment thereof which starts with a codon encoding the first amino acid of the constant part of the light chain and ends with a codon encoding the last amino acid of the constant part or fragment thereof followed by a non-sense codon.

**25.** A DNA construct according to Claim 24 in which the first part encodes a variable domain having an amino acid sequence substantially identical to the amino acid sequence as shown in Seq. Id. No 3 or in Seq. Id. No 4 and the second part encodes the constant part of the human $\kappa$ chain.

**26.** An expression vector comprising a DNA construct according to any one of claims 20-25 in operative association with a suitable promoter.

**27.** A host cell transformed with a vector according to Claim 26.

**28.** A process for the preparation of an LPS binding molecule according to any one of Claims 6-19 comprising the steps of culturing the host cell of Claim 27 and isolating the expressed protein.

29. An LPS binding molecule according to any one of Claims 6-19 for use as a pharmaceutical.

30. A pharmaceutical composition comprising an LPS binding molecule according to any one of Claims 6-19 in association with a pharmaceutically acceptable diluent or carrier.


**Patentansprüche**

1. Monoklonaler Antikörper, der ein Epitop in der Kernregion des LPS-Moleküls erkennt und der eine Kreuzschutzwirkung gegen Endotoxämie, die durch mindestens zwei unterschiedliche gram-negative Bakterienstämme mit unterschiedlichen Kernstrukturen hervorgerufen wird, entfaltet, wobei der monoklonale Antikörper eine konstante Region humanen Ursprungs, eine variable Gerüstregion humanen oder nicht-humanen Ursprungs und eine hypervariable Region nicht-humanen Ursprungs umfaßt.

2. Monoklonaler Antikörper nach Anspruch 1, der ein Epitop erkennt, das vollständig in der Rc-Kernstruktur von E. coli vorhanden ist, das auch im vollständigen Kern vorhanden ist.

3. Monoklonaler Antikörper nach Anspruch 1 oder 2, wobei die hypervariable Region murinen Ursprungs ist.

4. Monoklonaler Antikörper nach einem der vorstehenden Ansprüche, der vom IgG-Isotyp ist.

5. Hybridom-Zellinie, die einen monoklonalen Antikörper nach einem der vorstehenden Ansprüche erzeugt.

6. LPS-bindendes Molekül, das mindestens eine Antigenbindungsstelle umfaßt, die mindestens eine Domäne umfaßt, die in Folge die hypervariablen Regionen hCDR1, hCDR2 und hCDR3 umfaßt;

   wobei hCDR1 die Aminosäuresequenz Asp Tyr Tyr Met Thr aufweist;
   wobei hCDR2 die Aminosäuresequenz Leu Ile Arg Asn W Arg Asn Gly Asp Thr Ala Glu Tyr Ser Ala Ser Val X aufweist;
   wobei W Lys oder Tyr bedeutet und X Lys oder Arg bedeutet;
   wobei hCDR3 die Aminosäuresequenz Gln Gly Arg Gly Tyr Thr Leu Asp Tyr aufweist;
   und direkte Äquivalente davon.

7. Antikörper mit einer einzigen Domäne gemäß Anspruch 6, umfassend in Folge die hypervariablen Regionen hCDR1, hCDR2 und hCDR3, die mit murinen oder humanen H-Ketten-Gerüstregionen unter Bildung einer isolierten variablen H-Kettendomäne assoziiert sind.

8. LPS-bindendes Molekül gemäß Anspruch 6, umfassend mindestens eine Antigenbindungsstelle, die folgendes umfaßt:

   a) eine erste Domäne, die in Folge die hypervariablen Regionen hCDR1, hCDR2 und hCDR3 gemäß der Definition in Anspruch 6 umfaßt; und
   b) eine zweite Domäne, die in Folge die hypervariablen Regionen lCDR1, lCDR2 und lCDR3 umfaßt;

   wobei lCDR1 die Aminosäuresequenz Arg Ala Y Z Asn Ile Asn Ile Trp Leu Ser aufweist;
   wobei Y Ser oder Arg bedeutet und Z Gln oder Leu bedeutet;
   wobei lCDR2 die Aminosäuresequenz Lys Ala Ser Asn Leu His Thr aufweist;
   wobei lCDR3 doe Aminosäuresequenz Leu Gln Gly Gln Ser Tyr Pro Arg Thr aufweist;
   und direkte Äquivalente davon.

9. LPS-bindendes Molekül gemäß Anspruch 8 oder 9, bei dem die hypervariablen Regionen mit murinen oder humanen Gerüstregionen assoziiert sind.

10. LPS-bindendes Molekül gemäß Anspruch oder 9, bei dem die erste und zweite Domän Teil einer einzelnen gemeinsamen Peptidkette sind.

**11.** Einzelkettiger Antikörper gemäß Anspruch 10, bei dem es sich bei der ersten und zweiten Domäne um eine variable Ig-H-Kettendomäne bzw. um eine variable Ig-L-Kettendomäne handelt, die kovalent durch einen Peptidlinker, der aus 10 bis 30 Aminosäuren besteht, gebunden sind.

**12.** LPS-bindendes Molekül gemäß Anspruch 8 oder 9, bei dem die erste Domäne Teil einer H-Kette mindestens eines Fragments eines Ig-Moleküls ist und die zweite Kette Teil einer L-Kette mindestens eines Fragments eines Ig-Moleküls ist.

**13.** LPS-bindendes Molekül gemäß Anspruch 12, bei dem es sich um ein vollständiges Ig-Molekül handelt.

**14.** Ig-Molekül gemäß Anspruch 13, das vom IgG-Isotyp ist.

**15.** Ig-Molekül gemäß Anspruch 13 oder 14, bei dem es sich um ein murines Molekül handelt.

**16.** Ig-Molekül gemäß Anspruch 13 oder 14, bei dem die variablen Domänen murin und die konstanten Domänen human sind.

**17.** Ig-Molekül gemäß Anspruch 13 oder 14, bei dem die Gerüstregionen und die konstanten Regionen human sind.

**18.** Ig-Molekül gemäß Anspruch 15 oder 16, bei dem die variable H-Kettendomäne eine Aminosäuresequenz aufweist, die im wesentlichen identisch mit Seq. Id. No. 1 oder alternativ mit Seq. Id. No. 2 ist und die variable L-Kettendomäne eine Aminosäuresequenz aufweist, die im wesentlichen identisch mit der von Seq. Id. No. 3 oder alternativ mit Seq. Id. No. 4 ist.

**19.** Ig-Molekül gemäß Anspruch 18 unter Rückbeziehung auf Anspruch 16, bei dem die konstante H-Kettendomäne vom humanen Typ $\gamma_1$ und die konstante L-Kettendomäne vom humanen Typ $\kappa$ ist.

**20.** DNA-Konstrukt, das für eine Aminosäuresequenz kodiert, die in Folge die hypervariablen Regionen hCDR1, hCDR2 und hCDR3 gemäß den Angaben in Anspruch 6 umfaßt.

**21.** DNA-Konstrukt, kodierend für eine H-Kette oder ein Fragment davon, umfassend

a) einen ersten Teil, der für eine variable Domäne kodiert, die abwechselnd Gerüst- und hypervariable Regionen umfaßt, wobei es sich bei den hypervariablen Regionen in Folge um hCDR1, hCDR2 und hCDR3 handelt, deren Aminosäuresequenzen in Anspruch 6 angegeben sind, wobei dieser erste Teil mit einem Codon beginnt, das für die erste Aminosäure der variablen Domäne kodiert, und mit einem Codon endet, das für die letzte Aminosäure der variablen Domäne kodiert, und
b) einen zweiten Teil, der für einen konstanten H-Kettenteil oder ein Fragment davon kodiert, das mit einem Codon beginnt, das für die erste Aminosäure des konstanten Teils der H-Kette kodiert, und mit einem Codon endet, das für die letzte Aminosäure des konstanten Teils oder eines Fragments davon kodiert, wonach ein Nonsense-Codon folgt.

**22.** DNA-Konstrukt gemäß Anspruch 21, bei dem der erste Teil für eine variable Domäne mit einer Aminosäuresequenz, die im wesentlichen identisch mit der in Seq. Id. No. 1 oder alternativ No. 2 dargestellten Aminosäuresequenz ist, und der zweite Teil für den konstanten Teil der humanen $\gamma_1$-Kette kodiert.

**23.** DNA-Konstrukt, kodierend für eine Aminosäuresequenz, die in Folge die hyypervariablen Regionen ICDR1, ICDR2 und ICDR3 gemäß den Angaben in Anspruch 8 umfaßt.

**24.** DNA-Konstrukt, kodierend für eine L-Kette oder ein Fragment davon und umfassend:

a) einen ersten Teil, der für eine variable Domäne kodiert, die abwechselnd Gerüst- und hypervariable Regionen umfaßt, wobei es sich bei den hypervariablen Regionen in Folge um ICDR1, ICDR2 und ICDR3 handelt, deren Aminosäuresequenzen in Seq. Id. No. 3 oder in Seq. Id. No. 4 dargestellt sind, wobei der erste Teil mit einem Codon beginnt, das für die erste Aminosäure der variablen Domäne kodiert, und mit einem Codon endet, das für die letzte Aminosäure der variablen Domäne kodiert, und
b) einen zweiten Teil, der für einen konstanten L-Kettenteil oder ein Fragment davon kodiert, das mit einem Codon beginnt, das für die erste Aminosäure des konstanten Teils der L-Kette kodiert, und mit einem Codon

EP 0 576 439 B1

endet, das für die letzte Aminosäure des konstanten Teils oder eines Fragments davon kodiert, wonach ein nonsense-Codon folgt.

**25.** DNA-Konstrukt gemäß Anspruch 24, bei dem der erste Teil für eine variable Domäne mit einer Aminosäuresequenz, die im wesentlichen identisch mit der in Seq. Id. No. 3 oder in Seq. Id. No. 4 dargestellten Aminosäuresequenz ist, kodiert und der zweite Teil für den konstanten Teil der humanen κ-Kette kodiert.

**26.** Expressionsvektor, umfassend ein DNA-Konstrukt gemäß einem der Ansprüche 20-25 in funktioneller Assoziation mit einem geeigneten Promotor.

**27.** Wirtszelle, transformiert mit einem Vektor gemäß Anspruch 26.

**28.** Verfahren zur Herstellung eines LPS-bindenden Moleküls gemäß einem der Ansprüche 6-19, umfassend die Stufen der Züchtung der Wirtszelle von Anspruch 27 und die Isolierung des exprimierten Proteins.

**29.** LPS-bindendes Molekül gemäß einem der Ansprüche 6-19 zur Verwendung als Arzneimittel.

**30.** Arzneimittelzusammensetzung, umfassend ein LPS-bindendes Molekül gemäß einem der Ansprüche 6-19 in Verbindung mit einem pharmazeutisch verträglichen Verdünnungsmittel oder Trägerstoff.

**Revendications**

**1.** Anticorps monoclonal qui reconnaît un épitope dans la région du noyau de la molécule de lipopolysaccharide (LPS) et qui fournit une protection croisée contre une endotoxémie provoquée par au moins deux souches bactériennes Gram-négatives différentes ayant des structures de noyau différentes, ledit anticorps monoclonal ayant une région constante d'origine humaine, une région de cadre variable d'origine humaine ou non humaine, et une région hypervariable d'origine non humaine.

**2.** Anticorps monoclonal suivant la revendication 1, qui reconnaît un épitope qui est complètement présent dans la structure de noyau Rc de *E. coli* et est aussi présent dans le noyau complet.

**3.** Anticorps monoclonal suivant les revendications 1 ou 2, dans lequel la région hypervariable est d'origine murine.

**4.** Anticorps monoclonal suivant l'une quelconque des revendications précédentes qui est d'isotype IgG.

**5.** Lignée cellulaire d'hybridome produisant un anticorps monoclonal suivant l'une quelconque des revendications précédentes.

**6.** Molécule de fixation de LPS qui comprend au moins un site de fixation d'antigène comprenant au moins un domaine qui comprend en séquence, les régions hypervariables hCDR1, hCDR2 et hCDR3;

ladite hCDR1 ayant la séquence d'acides aminés Asp Tyr Tyr Met Thr;
ladite hCDR2 ayant la séquence d'acides aminés Leu Ile Arg Asn W Arg Asn Gly Asp Thr Ala Glu Tyr Ser Ala Ser Val X, dans laquelle W est Lys ou Tyr et X est Lys ou Arg;
ladite hCDR3 ayant la séquence d'acides aminés Gln Gly Arg Gly Tyr Thr Leu Asp Tyr;

et des équivalents directs de celles-ci.

**7.** Anticorps à domaine unique suivant la revendication 6, comprenant en séquence, les régions hypervariables hCDR1, hCDR2 et hCDR3 associées à des régions de cadre de chaînes lourdes humaine ou murine de façon à former un domaine variable isolé de chaîne lourde.

**8.** Molécule de fixation de LPS suivant la revendication 6, comprenant au moins un site de fixation d'antigène comprenant :

(a) un premier domaine comprenant en séquence, les régions hypervariables hCDR1, hCDR2 et hCDR3 tels qu'elles sont définies dans la revendication 6 et,

(b) un second domaine comprenant en séquence, les régions hypervariables ICDR1, ICDR2 et ICDR3 ;

ladite ICDR1 ayant la séquence d'acides aminés Arg Ala Y Z Asn Ile Asn Ile Trp Leu Ser; dans laquelle Y est Ser ou Arg et Z est Gln ou Leu;
ladite ICDR2 ayant la séquence d'acides aminés Lys Ala Ser Asn Leu His Thr;
ladite ICDR3 ayant la séquence d'acides aminés Leu Gln Gly Gln Ser Tyr Pro Arg Thr;

et des équivalents directs de celles-ci.

9. Molécule de fixation de LPS suivant la revendication 8, dans laquelle les régions hypervariables sont associées à des régions de cadre murines ou humaines.

10. Molécule de fixation de LPS suivant les revendications 8 ou 9, dans laquelle les premier et second domaines font partie d'une chaîne peptidique commune unique.

11. Anticorps à chaîne unique suivant la revendication 10, dans lequel les premier et second domaines sont respectivement un domaine variable de chaîne lourde d'Ig et un domaine variable de chaîne légère d'Ig, et sont liés par covalence par une séquence de liaison peptidique consistant en 10 à 30 acides aminés.

12. Molécule de fixation de LPS suivant les revendications 8 ou 9, dans laquelle le premier domaine fait partie d'une chaîne lourde d'au moins un fragment d'une molécule d'Ig, et le second domaine fait partie d'une chaîne légère d'au moins un fragment d'une molécule d'Ig.

13. Molécule de fixation de LPS suivant la revendication 12, qui est une molécule d'Ig complète.

14. Molécule d'Ig suivant la revendication 13, qui est d'isotype Ig.

15. Molécule d'Ig suivant les revendications 13 ou 14, qui est murine.

16. Molécule d'Ig suivant les revendications 13 ou 14, dans laquelle les domaines variables sont murins et les domaines constants sont humains.

17. Molécule d'Ig suivant les revendications 13 ou 14, dans laquelle les régions de cadre et les domaines constants sont humains.

18. Molécule d'Ig suivant les revendications 15 ou 16, dans laquelle le domaine variable de la chaîne lourde a une séquence d'acides aminés pratiquement identique à celle qui est donnée dans la Seq. Id. No 1 ou dans une variante dans la Seq. Id. No 2 et le domaine variable de la chaîne légère a une séquence d'acides aminés pratiquement identique à celle qui est donnée dans la Seq. Id. No 3 ou dans une variante dans la Seq. Id. No 4.

19. Molécule d'Ig suivant la revendication 18 lorsque celle-ci est en relation avec la revendication 16, dans laquelle le domaine constant de la chaîne lourde est de type γ1 humain et le domaine constant de la chaîne légère est de type κ humain.

20. Construction d'ADN codant pour une séquence d'acides aminés comprenant en séquence les régions hypervariables hCDR1, hCDR2 et hCDR3 définies dans la revendication 6.

21. Construction d'ADN codant une chaîne lourde ou un fragment de celle-ci et comprenant :

(a) une première partie qui code un domaine variable comprenant alternativement des régions de cadre et hypervariables, lesdites régions hypervariables étant en séquence hCDR1, hCDR2 et hCDR3, dont les séquences d'acides aminés sont définies dans la revendication 6; cette première partie commençant avec un codon codant le premier acide aminé du domaine variable et finissant avec un codon codant le dernier acide aminé du domaine variable, et
(b) une seconde partie codant une partie constante de la chaîne lourde ou un fragment de celle-ci, qui commence avec un codon codant le premier acide aminé de la partie constante de la chaîne lourde et finit avec un codon codant le dernier acide aminé de la partie constante ou d'un fragment de celle-ci, suivi d'un codon non sens.

22. Construction d'ADN suivant la revendication 21, dans laquelle la première partie code un domaine variable ayant une séquence d'acides aminés pratiquement identique à la séquence d'acides aminés telle qu'elle est montrée dans la Seq. Id. No 1 ou dans une variante dans la Seq. Id. No 2 et la seconde partie code la partie constante de la chaîne γ1 humaine.

23. Construction d'ADN codant pour une séquence d'acides aminés comprenant en séquence les régions hypervariables JCDR1, ICDR2 et ICDR3 définies dans la revendication 8.

24. Construction d'ADN codant une chaîne légère ou un fragment de celle-ci et comprenant :

(a) une première partie qui code un domaine variable comprenant alternativement des régions de cadre et hypervariables, lesdites régions hypervariables étant en séquence ICDR1, ICDR2 et ICDR3, dont les séquences d'acides aminés sont montrées dans la Seq. Id. No 3 ou dans la Seq. Id. No 4; cette première partie commençant avec un codon codant le premier acide aminé du domaine variable et finissant avec un codon codant le dernier acide aminé du domaine variable, et

(b) une seconde partie codant une partie constante de la chaîne légère ou un fragment de celle-ci, qui commence avec un codon codant le premier acide aminé de la partie constante de la chaîne légère et finit avec un codon codant le dernier acide aminé de la partie constante ou d'un fragment de celle-ci, suivi d'un codon non sens.

25. Construction d'ADN suivant la revendication 24, dans laquelle la première partie code un domaine variable ayant une séquence d'acides aminés pratiquement identique à la séquence d'acides aminés qui est montrée dans la Seq. Id. No 3 ou dans la Seq. Id. No 4 et la seconde partie code la partie constante de la chaîne κ humaine.

26. Vecteur d'expression comprenant une construction d'ADN suivant l'une quelconque des revendications 20 à 25 en association opérative avec un promoteur convenable.

27. Cellule hôte transformée avec un vecteur suivant la revendication 26.

28. Procédé pour la préparation d'une molécule de fixation de LPS suivant l'une quelconque des revendications 6 à 19 comprenant les étapes de culture de la cellule hôte suivant la revendication 27 et l'isolement de la protéine exprimée.

29. Molécule de fixation de LPS suivant l'une quelconque des revendications 6 à 19 utile en tant que produit pharmaceutique.

30. Composition pharmaceutique comprenant une molécule de fixation de LPS suivant l'une quelconque des revendications 6 à 19 en association avec un support ou diluant acceptable du point de vue pharmaceutique.

EP 0 576 439 B1

# FIG. 1

# FIG. 2

# FIG. 3a

# FIG. 3b

# FIG. 4a

St 1 2 3 4 5 6 7 8 St

# FIG. 4b

St  1  2  3  4  5  6  7  8  St

FIG. 4c

St 1 2 3 4 5 6 7 8 St

FIG. 4d

# FIG. 5

RESTRICTION SITES

## FIG. 6a

## FIG. 6b

FIG. 7

EP 0 576 439 B1